(19) Europäisches Patentamt
European Patent Office
Office européen des brevets

(11) EP 3 166 916 B1

(12) **FASCICULE DE BREVET EUROPEEN**

(45) Date de publication et mention de la délivrance du brevet:
**24.02.2021 Bulletin 2021/08**

(21) Numéro de dépôt: **15736636.0**

(22) Date de dépôt: **17.06.2015**

(51) Int Cl.:
***C07C 37/055*** *(2006.01)* ***C07C 39/08*** *(2006.01)*
***C05F 11/00*** *(2006.01)* ***C08H 7/00*** *(2011.01)*

(86) Numéro de dépôt international:
**PCT/IB2015/054567**

(87) Numéro de publication internationale:
**WO 2016/005836 (14.01.2016 Gazette 2016/02)**

(54) **PROCÉDÉ DE DÉPOLYMÉRISATION DE LA LIGNINE**

VERFAHREN ZUR DEPOLYMERISIERUNG VON LIGNIN

METHOD OF DEPOLYMERIZING LIGNIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.07.2014 FR 1456637**

(43) Date de publication de la demande:
**17.05.2017 Bulletin 2017/20**

(73) Titulaire: **Commissariat à l'Énergie Atomique et aux Énergies Alternatives
75015 Paris (FR)**

(72) Inventeurs:
- **FEGHALI, Elias
Springfield, 3015 Rotorua (NZ)**
- **CANTAT, Thibault
92130 Issy Les Moulineaux (FR)**

(74) Mandataire: **Gevers & Orès
Immeuble le Palatin 2
3 Cours du Triangle
CS 80165
92939 Paris La Défense Cedex (FR)**

(56) Documents cités:
**WO-A2-2011/003029**

- **JIANFENG ZHANG ET AL: "Reductive Degradation of Lignin and Model Compounds by Hydrosilanes", ACS SUSTAINABLE CHEMISTRY & ENGINEERING, vol. 2, no. 8, 3 juillet 2014 (2014-07-03) , pages 1983-1991, XP055171789, ISSN: 2168-0485, DOI: 10.1021/sc500302j**
- **ELIAS FEGHALI ET AL: "Unprecedented organocatalytic reduction of lignin model compounds to phenols and primary alcohols using hydrosilanes", CHEMICAL COMMUNICATIONS, vol. 50, no. 7, 1 janvier 2014 (2014-01-01), pages 862-865, XP055171824, ISSN: 1359-7345, DOI: 10.1039/C3CC47655C cité dans la demande**
- **Jianfeng Zhang ET AL: "Reductive Degradation of Lignin and Model Compounds by Hydrosilanes", ACS Sustainable Chemistry & Engineering, vol. 2, no. 8, 4 August 2014 (2014-08-04), pages 1983-1991, XP055171801, ISSN: 2168-0485, DOI: 10.1021/sc500302j**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 166 916 B1

Printed by Jouve, 75001 PARIS (FR)

## Description

La présente invention concerne un procédé de dépolymérisation de la lignine et l'utilisation de ce procédé dans la fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais.

Elle concerne également un procédé de fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais, comprenant une étape de dépolymérisation de la lignine par le procédé selon l'invention.

Le bois est composé de trois constituants majeurs : la cellulose, l'hémicellulose et la lignine. La cellulose et l'hémicellulose sont déjà valorisées dans l'industrie, en particulier dans l'industrie papetière. Cette utilisation génère chaque année plusieurs millions de tonnes de sous-produits riches en lignine qui sont utilisés comme combustibles de faible capacité calorifique pour fournir chaleur et énergie aux procédés de production du papier. En parallèle, une quantité minime de la lignine est isolée par extraction directe à partir de plantes (F. G. Calvo-Flores et J. A. Dobado, ChemSusChem. 2010, 3, pages 1227-1235).

La lignine est la substance la plus abondante en terme de source de groupements aromatiques dans la nature et le plus grand contributeur au sol de la matière organique (S. Y. Lin, in Methods in Lignin Chemistry, Springer Series in Wood Science (Ed.: C. W. Dence), Springer, Berlin 1992). Elle résulte de la polymérisation radicalaire de trois monomères nommés monolignols : l'alcool p-coumarilyque, l'alcool coniférylique, et l'alcool sinapylique, qui après une polymérisation par déshydrogénation avec la péroxydase donnent respectivement les résidus p-hydroxyphényle (H), guaiacyle (G), et syringyl (S) comme illustré dans la Figure 1 (R. Vanholme, K. Morreel, J. R. W. Boerjan, Curr. Opin. Plant Biol. 2008, 11, pages 278-285).

La complexité ainsi que la diversité de la structure de la lignine est largement dépendante de son origine. En se basant sur la taxonomie végétale, il a été proposé que la lignine issue des gymnospermes (appelés « bois tendre » ou « softwood » en anglais) présente plus de résidus G, que celle issue des angiospermes (appelés « bois dur » ou hardwood en anglais), qui contient un mélange de résidus G et S, et la lignine issue des plantes herbacées contient un mélange des trois résidus aromatiques H, G et S. Une technique de classification plus rigoureuse a été de se baser sur une approche chimique dans laquelle les lignines sont classées selon l'abondance des motifs G, H et S dans le polymère. Quatre grands groupes de lignine ont ainsi été identifiés : le type G, le type-GS, le type HGS et le type HG (F. G. Calvo-Flores et J. A. Dobado, ChemSusChem. 2010, 3, pages 1227-1235).

Quel que soit le type de lignine, ce bio-polymère se caractérise par une grande hétérogénéité chimique et est constitué d'unités propyl-phénole liées entre elles par l'intermédiaire de divers types de liaisons C-O et C-C de type aryle éther, aryle glycérol et β-aryle éther. La Figure 2 montre la structure de la lignine proposée par E. Adler, Wood Sci. Technol. 1977, 11, page 169.

Les liaisons éthers représentent environ les deux tiers des liaisons. Plus spécifiquement, les liaisons de type β-O-4 et α-O-4, qui font partie des alkylaryle éthers, sont les plus abondantes. Typiquement, la lignine des angiospermes (bois dur ou hardwood en anglais) contient 60% de liaisons de type β-O-4 et 6-8% de type α-O-4, et la lignine issue des gymnospermes (bois tendre ou softwood en anglais) contient 46% de liaisons de type β-O-4 et 6-8% de type α-O-4. Bien que la proportion de ces liaisons varie considérablement d'une espèce à une autre, des valeurs typiques extraites de M. P. Pandey, C. S. Kim, Chem. Eng. Technol., 2011, 34, 29, ont été répertoriées dans le tableau de la Figure 3.

Les structures chimiques des types de liaisons les plus abondantes présentes dans la lignine, sont représentées en Figure 4.

La lignine représente le plus grand réservoir renouvelable de composés aromatiques disponibles. En raison de sa forte teneur en aromatiques, la lignine a un grand potentiel pour fonctionner comme une alternative aux ressources fossiles non renouvelables pour la production de produits chimiques aromatiques à haute valeur ajoutée, c'est-à-dire des produits dont la transformation augmente de façon considérable leur valeur commerciale. A titre de produits chimiques aromatiques à haute valeur ajoutée, on peut citer, par exemple, le 4-propylbenzène-1,2-diol (à 3700 $/kg) ou le 4-(3-hydroxypropyl)-1,2-benzènediol (à 3100 $/kg). Ainsi, la valorisation de la lignine implique sa conversion en produits aromatiques précieux et utiles via sa dépolymérisation. Toutefois, en raison de sa structure amorphe, polymérique et basée sur des liaisons éthers fortes, sa dépolymérisation pour produire des molécules utilisables représente un défi. En outre, les lignines sont structurellement très diversifiées et, en fonction de la source végétale utilisée, elles contiennent des proportions différentes des trois monomères de base (l'alcool p-coumarylique, l'alcool coniférylique et l'alcool sinapylique).

Le développement d'un procédé de dépolymérisation par le clivage des liaisons éthers contribuera donc d'une façon significative à la valorisation de la lignine. Or, la dépolymérisation directe de la lignine est difficile, car sa structure est hautement fonctionnalisée et ramifiée et son encombrement stérique peut limiter l'accès du catalyseur aux sites actifs. D'autre part, l'hétérogénéité chimique de la lignine qui est due à la présence de plusieurs résidus G, H, S présents

à des taux variables selon la nature de la plante et à la présence de divers types de liaisons C-O et C-C de type aryle éther, aryle glycérol et β-aryle éther, complique l'obtention de produits chimiques purs lors de la transformation de la lignine.

Compte tenu de la difficulté à procéder à une dépolymérisation directe de la lignine, les scientifiques ont synthétisé des modèles, chimiquement purs, représentatifs des liens éthers présents dans la lignine, pour en étudier la réactivité (J. Zakzeski, P. C. A. Bruijnincx, A. L. Jongerius and B. M. Weckhuysen, Chem Rev., 2010, 110, page 3552). La majorité des études visant la dépolymérisation de la lignine s'est focalisée sur ces modèles et n'a pas été mise en œuvre sur la structure complexe de lignines naturelles. Des exemples de clivage de liaisons C-O du motif β-O-4 sur des modèles de lignine en utilisant la catalyse redox ou réductrice sont donnés ci-après.

- Bergman, Ellman *et al.* (J. M. Nichols, L. M. Bishop, R. G. Bergman, J. A. Ellman, J. Am. Chem. Soc. 2010, 132, pages 12554-12555) ont développé une réaction de clivage redox de liaison C-O catalysé au ruthénium. Les modèles des motifs β-O-4 de la lignine ont été clivés avec des rendements isolés allant de 62 à 98%. La réaction se réalise selon un mécanisme tandem de déshydrogénation de l'a-alcool suivi d'un clivage réducteur de l'aryle éther.
  D'autre part, James *et al.* (A. Wu, B. O. Patrick, E. Chung and B. R. James, Dalton Trans., 2012, 41, page 11093) ont montré qu'un complexe de ruthénium est capable de catalyser l'hydrogénolyse directe de l'équivalent cétone du motif β-O-4 avec de l'hydrogène gazeux. Cependant, les auteurs ont observés que des modèles du motif β-O-4 contenant la fonction γ-OH, n'étaient pas réactifs.
  Récemment, Leitner *et al.* (T. vom Stein,T. Weigand, C. Merkens, Jurgen Klankermayer. W. Leitner, ChemCatChem, 2013, 5, pages 439 - 441) ont décrit une réaction de clivage redox de liaisons C-O du motif β-O-4, par le biais d'un transfert d'hydrogène intramoléculaire. Cette réaction met en jeu un catalyseur à base de ruthénium (métal noble et cher), un ligand triphos (ligand également très cher) et des températures élevées (chauffage à 135°C). De plus, selon le modèle du motif β-0-4 utilisé, la mise en œuvre de la réaction peut s'avérer plus ou moins simple.

- Un catalyseur de vanadium a été utilisé par Toste *et al.* (S. Son and F. D. Toste, Angew. Chem, Int, Ed. 2010, 49, pages 3791-3794) pour le clivage de liaisons C-O du motif β-0-4 et la formation d'aryle énones. Cette transformation redox est effectuée dans l'acétate d'éthyle à 80°C. La charge de catalyseur est de 10% en moles et après 24 heures, la réaction peut atteindre jusqu'à 95% de conversion du modèle de lignine de départ en aryle énone. S'agissant d'une réaction redox, les produits obtenus sont en général très oxygénés et donc pauvres en énergie.
  Plus récemment, le même groupe (J. M. W. Chan, S. Bauer, H. Sorek, S. Sreekumar, K. Wang, F. D, Toste, ACS Catal., 2013, 3, pages 1369-1377) a démontré l'applicabilité de ce procédé de clivage redox à la dégradation de la lignine extraite de *Miscanthus giganteus* (herbe aux éléphants). Les résultats des études CPG et RMN 2D de la dégradation de la lignine dioxasolv et acetosolv ressemblaient aux données obtenues avec les modèles de lignine, ce qui confirme la sélectivité du procédé pour les liaisons β-O-4. Par ailleurs, seul le *Miscanthus giganteus,* qui est une herbe, a été essayé et pas le bois. Enfin, les auteurs ont pu identifier et quantifier par GC/MS, des composés phénoliques volatils (comme la vanilline, l'acide vanillique, l'acide syringique et le syringaldéhyde) produits dans la réaction. Néanmoins, aucun produit chimique pur n'a pu être isolé à partir de ce procédé et des mélanges partiellement caractérisés ont été obtenus.

- En 2011, une procédé sélectif d'hydrogénolyse des liaisons C-O aromatiques dans les alkylaryle éthers et diaryles éthers a été développée par Sergeev et Hartwig (A. G. Sergeev and J. F. Hartwig, Science, 2011, 332, page 439). Ce procédé permet la formation sélective des arènes et des alcools à partir des modèles de lignine et en utilisant un complexe nickel-carbène soluble. La réaction se réalise dans le m-xylène, sous 1 bar d'hydrogène et à des températures allant de 80 à 120°C. L'utilisation de ce procédé permet le clivage de modèles de liaison 4-O-5 (diaryle éther), pour donner l'anisole, le benzène, et des phénols avec des rendements modérés. De même, l'hydrogénolyse des modèles de motif α-O-4 de lignine à 80°C sous 1 bar d'hydrogène donne le 3,4 diméthoxytoluène et 2-méthoxyphénol avec des rendements quasi quantitatifs. Le clivage du modèle β-O-4 dans des conditions basiques se réalise sans la présence de catalyseur et fournit le gaïacol à 89% de rendement mais en mélange avec de nombreux autres produits.

- Les groupes de Toste, Ellman et Hartwig ont regroupé leurs résultats sur la réduction de la lignine et de ses modèles en catalyse homogène dans la demande internationale WO2011003029. Les précurseurs utilisés sont des dérivés de vanadium, de ruthénium et de rhodium. Seuls les complexes à base de vanadium et de ruthénium ont été utilisés pour la dépolymérisation redox de la lignine extraite de *Miscanthus giganteus.* Néanmoins, aucun produit chimique pur n'a pu être isolé ni identifié par ce procédé et des mélanges partiellement caractérisés ont été obtenus.

- En 2009, Ragauskas *et al.* (M. Nagy, K. David, G. J. P. Britovsek and A. J. Ragauskas, Holzforschung, 2009, 63, page 513) ont réussi à dépolymériser la lignine éthanol organosolv (EOL) (soluble dans l'éthanol) issue du pin dans

des conditions réductrices. Dans cette étude, des catalyseurs hétérogènes classiques ainsi que des nouveaux catalyseurs homogènes ont été employés pour le clivage de liaison diaryle éthers et dialkyle éthers.
En employant les conditions d'hydrogenolyse : 5 MPa $H_2$ ; 175°C ; 20 heures, le catalyseur de ruthénium augmente efficacement la solubilité de la lignine (solubilité jusqu'à 96%) et contribue à sa dégradation. Une diminution de l'ordre de 10% à 20% de la masse molaire moyenne en masse (Mw) a été obtenue (Mw =1900-2100 g/mol), ce qui correspond à un dégrée de polymérisation (DP) de 10 à 11 unités de monomères (L.B. Davin, L.B., N. G. Lewis, Curr. Opin. Biotechnol., 2005, 16, pages 407 - 415). D'autre part, d'après les auteurs, l'hydrogenolyse des groupements diaryles éthers et alkylaryle éthers est accompagnée d'une réaction d'hydrogénation simultanée du cycle aromatique. Enfin, l'identification ainsi que la formation détaillée des produits de réaction et des voies de clivage n'ont pas été élucidés.

- En 2013, la première réduction organocatalytique des composés modèles de la lignine a été décrite par Féghali et Cantat (E. Feghali, T. Cantat, Chem. Commun., 2014, 50, pages 862-865). Ces derniers ont montré que le $B(C_6F_5)_3$ est un catalyseur d'hydrosilylation efficace et sélectif pour le clivage réducteur des liaisons alkylaryle éthers et en particulier des modèles de motifs α-O-4 et β-O-4. De plus, la réduction se réalise dans des conditions douces (température ambiante de 2 à 16 heures), et peut être menée avec une source d'hydrure stable à l'air et peu coûteuse comme le polyméthylhydrosiloxane (PMHS) et le tétraméthyldisilazane (TMDS). Néanmoins, ce procédé n'a pu être extrapolé à la dépolymérisation directe de la lignine.

-En 2014, une dégradation réductrice d'une lignine Kraft et de lignine de bois mou par un mélange de silanes et de boranes, permettant l'obtention de molécules ayant un $M_w$ égal à 465 g/mol à été décrit par Jianfeng Zhang et al (J. Zheng, Y. Chen, M.A. Brook, Acs Sustainable Chemistry and Engineering, 2014, 2, 1983-1991, published on July 3, 2014).

Etant donné la structure polymérique complexe, hétérogène et fortement encombrée de la lignine qui complique sa dépolymérisation, les procédés de dépolymérisation développés dans la littérature et décrits ci-dessus se réalisent, en général, dans des conditions drastiques de température et de pression et mettent en œuvre des métaux en quantités catalytiques élevées. Par ailleurs, ces procédés ont été développés sur des modèles chimiquement purs, et rares sont ceux qui ont pu être extrapolés sur la réduction de la lignine. En fait, seuls les procédés de Ragauskas *et al.* (M. Nagy, K. David, G. J. P. Britovsek and A. J. Ragauskas, Holzforschung, 2009, 63, page 513) et de Toste *et al.* (S. Son and F. D. Toste, Angew. Chem. Int. Ed. 2010, 49, pages 3791-3794) ont pu être extrapolés à la lignine. Les autres procédés n'ont pas fonctionné avec la lignine. La présence de plusieurs impuretés, notamment l'eau, l'oxygène ($O_2$), les molécules soufrées, les molécules phosphorées et les résidus de sucres peuvent désactiver le catalyseur. Ces impuretés peuvent provenir, par exemple, de la lignocellulose ou du procédé d'extraction de la lignine à partir de la lignocellulose.

Il existe donc un réel besoin d'un procédé permettant la dépolymérisation de la lignine et palliant les inconvénients de l'art antérieur.

En particulier, il existe un réel besoin d'un procédé permettant la dépolymérisation de la lignine, ledit procédé :

- présentant une grande efficacité se traduisant par une grande conversion de la lignine en molécules de plus petites tailles contenant 1 à 2 cycles aromatiques, et d'une grande sélectivité vis-à-vis de certaines liaisons de la lignine ;
- permettant de générer des molécules aromatiques de hautes valeurs ajoutées, en particulier des molécules contenant 1 à 2 cycles aromatiques ;
- simple à mettre en œuvre ;
- pouvant être mis en œuvre dans des conditions opératoires douces et industriellement intéressantes.

La présente invention a précisément pour but de répondre à ces besoins en fournissant un procédé de dépolymérisation de la lignine en molécules contenant de 1 à 2 cycles aromatiques, par clivage sélectif de la liaison carbone $sp^3$-oxygène des alkylaryle éthers du type β-O-4, α-O-4, β-5, β-1, β-β présents dans la lignine, caractérisé en ce que l'on fait réagir, en présence d'un catalyseur,

- une lignine avec un taux de soufre inférieur à 1,5% en masse, par rapport à la masse totale de la lignine, avec
- un composé silane de formule (I)

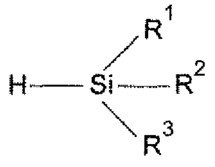

(I)

dans laquelle

- $R^1$, $R^2$ et $R^3$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe aryloxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
- $R^3$ est tel que défini ci-dessus et $R_1$ et $R_2$, pris ensemble avec l'atome de silicium auquel ils sont liés forment un hétérocycle silylé éventuellement substitué; et en ce que les molécules aromatiques contenant 1 à 2 cycles aromatiques présentent une masse molaire moyenne en poids inférieure à 1500 g/mol pour les molécules sous forme silylée ou de masse molaire moyenne en poids égale ou inférieure à 400g/mol sous forme non silylée, soit un degré de polymérisation entre 1 et 2 unités monomères ; et en ce que la lignine est extraite par les procédés organosolv.

Le procédé de dépolymérisation de la lignine selon l'invention est hautement sélectif vis-à-vis des liaisons carbone $sp^3$-oxygène des alkylaryle éthers présents dans la lignine et vise ainsi essentiellement les liaisons carbone $sp^3$-oxygène du type β-O-4, α-O-4, β-5, β-1, β-β. Sans vouloir être lié par la théorie, le clivage des liaisons carbone $sp^3$-oxygène au niveau des liaisons du type β-O-4, α-O-4 et β-β conduit à la fois à la dépolymérisation de la lignine et à la modification de sa structure, alors que le clivage des liaisons carbone $sp^3$-oxygène au niveau des liaisons du type β-5 et β-1, conduit à la modification de la structure de la lignine sans la coupure de la liaison entre deux unités monomériques successives.

Les liaisons carbone $sp^2$-oxygène des aryles éthers présents dans la lignine (essentiellement les liaisons du type 5-5 et 4-O-5) ainsi que toute autre liaison carbone $sp^2$-oxygène présente dans la lignine restent intactes lors du procédé de l'invention.

Le procédé de l'invention permet de s'affranchir des conditions réactionnelles drastiques de température et de pression utilisées traditionnellement dans la littérature pour la dépolymérisation de la lignine. Il permet également de réduire des coûts par l'utilisation des silanes de formule (I) qui sont stables à l'air et peu coûteux.

Le procédé de l'invention a l'avantage de permettre la dépolymérisation de la lignine conduisant à l'obtention de molécules contenant 1 à 2 cycles aromatiques de masse molaire moyenne en poids inférieure à 1500 g/mol pour les molécules sous forme silylée (molécules dont tous les atomes d'oxygène sont sous forme silylée O-Si) ou de masse molaire moyenne en poids égale ou inférieure à 400g/mol pour les molécules sous forme non silylées (c'est-à-dire des molécules dont toutes les liaisons O-Si ont été clivées par exemple par une hydrolyse), soit un degré de polymérisation inférieur à 3 unités monomères, de préférence entre 1 et 2 unités monomères. Les molécules aromatiques obtenus peuvent contenir des cycles aromatiques mono-, di- ou tri-oxygénés en fonction de l'abondance des motifs G, H et S dans la lignine utilisée. Les proportions des motifs G, H et S dépendent de l'espèce végétale dont provient la lignine ainsi que son procédé d'extraction. La masse molaire moyenne en poids des molécules obtenues et donc leur degré de polymérisation, peut être déterminée par toute méthode connue de l'homme du métier notamment par la chromatographie d'exclusion stérique (SEC pour Size Exclusion Chromatography en anglais).

Par ailleurs, le procédé de l'invention peut générer du méthane et d'hydrogène (de l'ordre de 7 à 15 % en masse de la masse de lignine introduite). Ces deux gaz peuvent éventuellement être utilisés comme combustibles pour fournir de l'énergie au procédé de l'invention.

Dans le procédé de l'invention, les composés silanes de formule (I) assurent le clivage par réduction des liaisons carbone $sp^3$-oxygène des alkylaryle éthers présents dans la lignine, en conditions catalytiques.

Les molécules aromatiques contenant 1 à 2 cycles aromatiques, de masse molaire moyenne en poids inférieure à 1500 g/mol pour les molécules sous forme silylée ou de masse molaire moyenne en poids égale ou inférieure à 450g/mol, de préférence égale ou inférieure à 400g/mol pour les molécules sous forme non silylées (soit un degré de polymérisation inférieur à 3 unités monomères, de préférence entre 1 et 2 unités monomères) sont ainsi obtenus avec une bonne productivité (de l'ordre de 20 à 99%, par exemple), et une très bonne sélectivité vis-à-vis des liaisons carbone $sp^3$-oxygène β-O-4, α-O-4, β-5, β-1 et β-β.

Dans le cadre de la présente invention, le rendement correspond à la quantité de molécules aromatiques contenant 1 à 2 cycles aromatiques, de masse molaire moyenne en poids inférieure à 1500 g/mol pour les molécules sous forme silylée ou de masse molaire moyenne en poids égale ou inférieure à 450g/mol, de préférence égale ou inférieure à 400g/mol (soit un degré de polymérisation inférieur à 3 unités monomères, de préférence entre 1 et 2 unités monomères) isolés par rapport à la quantité de lignine introduite initialement :

$$\text{Productivité} = \text{m(molécules aromatiques contenant 1 à 2 cycles aromatiques) / m(lignine)}$$

m étant la masse exprimée en gramme.

On entend par « alkyle », au sens de la présente invention, un radical carboné linéaire, ramifié ou cyclique, saturé, éventuellement substitué, comprenant 1 à 12 atomes de carbone. A titre d'alkyle saturé, linéaire ou ramifié, on peut citer par exemple les radicaux méthyle, éthyle, propyle, butyle, pentyle, hexyle, octyle, nonyle, décyle, undécyle, dodécanyle et leurs isomères ramifiés. Comme alkyle cylique, on peut citer les radicaux cyclopropyle, cyclobutyle, cyclopentyle, cyclohexyle, bicylco[2,1,1] hexyle, bicyclo[2,2,1]heptyle.

Par « alcényle » ou « alcynyle », on entend un radical carboné insaturé linéaire, ramifié ou cyclique, éventuellement substitué, ledit radical carboné insaturé comprenant 2 à 12 atomes de carbone comprenant au moins une double (alcényle) ou une triple liaison (alcynyle). A ce titre, on peut citer, par exemple, les radicaux éthylényle, propylényle, butényle, pentényle, hexényle, acétylényle, propynyle, butynyle, pentynyle, hexynyle et leurs isomères ramifiés. Comme alcényles cycliques insaturés, on peut citer par exemple le cyclopentényle, le cyclohexényle.

Les groupes alkyle, alcényle et alcynyle, au sens de l'invention, peuvent être éventuellement substitués par un ou plusieurs groupes hydroxyle ; un ou plusieurs groupes alkoxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle, avec les groupes alkoxy et aryle tels que définis dans le cadre de la présente invention.

Le terme « aryle » désigne de manière générale un substituant aromatique cyclique comportant de 6 à 20 atomes de carbone. Dans le cadre de l'invention le groupe aryle peut être mono- ou polycyclique. A titre indicatif, on peut citer les groupes phényle, benzyle et naphtyle. Le groupe aryle peut être éventuellement substitué par un ou plusieurs groupes hydroxyle, un ou plusieurs groupes alkoxy, un ou plusieurs groupes «siloxy», un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkoxy et alkyle tels que définis dans le cadre de la présente invention.

Le terme « hétéroaryle » désigne de manière générale un substituant aromatique mono- ou polycyclique comportant de 5 à 10 membres dont au moins 2 atomes de carbone, et au moins un hétéroatome choisi parmi l'azote, l'oxygène, le bore, le silicium, le phosphore et le soufre. Le groupe hétéroaryle peut être mono- ou poly- cyclique. A titre indicatif, on peut citer les groupes furyle, benzofuranyle, pyrrolyle, indolyle, isoindolyle, azaindolyle, thiophényle, benzothiophényle, pyridyle, quinolinyle, isoquinolyle, imidazolyle, benzimidazolyle, pyrazolyle, oxazolyle, isoxazolyle, benzoxazolyle, thiazolyle, benzothiazolyle, isothiazolyle, pyridazinyle, pyrimidilyle, pyrazinyle, triazinyle, cinnolinyle, phtalazinyle, quinazolinyle. Le groupe hétéroaryle peut être éventuellement substitué par un ou plusieurs groupes hydroxyle, un ou plusieurs groupes alkoxy, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes aryle, un ou plusieurs groupes alkyle, avec les groupes alkyle, alkoxy et aryle tels que définis dans le cadre de la présente invention.

Le terme « alkoxy » signifie un groupe alkyle, alcényle et alcynyle, tels que définis ci-dessus, liés par un atome d'oxygène (-O-alkyle, O-alcényle, O-alcynyle).

Le terme « aryloxy » signifie un groupe aryle tel que défini ci-dessus, lié par un atome d'oxygène (-O-aryle).

Le terme « hétérocycle » désigne de manière générale un substituant mono- ou polycyclique, comportant de 5 à 10 membres, saturé ou instauré, contenant de 1 à 4 hétéroatomes choisis indépendamment l'un de l'autre, parmi l'azote, l'oxygène, le bore, le silicium, le phosphore et le soufre. A titre indicatif, on peut citer les substituants morpholinyle, pipéridinyle, pipérazinyle, pyrrolidinyle, imidazolidinyle, imidazolinyle, pyrazolidinyle, tétrahydrofuranyle, tétrahydropyranyle, thianyle, oxazolidinyle, isoxazolidinyle, thiazolidinyle, isothiazolidinyle. L'hétérocycle peut être éventuellement substitué par un ou plusieurs groupes hydroxyle, un ou plusieurs groupes alkoxy, un ou plusieurs groupes aryle, un ou plusieurs atomes d'halogène choisis parmi les atomes de fluor, chlore, brome et iode, un ou plusieurs groupes nitro ($-NO_2$), un ou plusieurs groupes nitrile (-CN), un ou plusieurs groupes alkyle, avec les groupes alkyle, alkoxy et aryle tels que définis dans le cadre de la présente invention.

Par atome d'halogène, on entend un atome choisi parmi les atomes de fluor, chlore, brome et iode.

Par groupe « silylé », on entend un groupe de formule $[-Si(X)_3]$ dans lequel chaque X, indépendamment l'un de l'autre, est choisi parmi un atome d'hydrogène ; un ou plusieurs atomes d'halogène choisis parmi les atomes de fluor, chlore, brome ou iode ; un ou plusieurs groupes alkyle ; un ou plusieurs groupes alkoxy ; un ou plusieurs groupes aryle ; un ou plusieurs groupes siloxy ; avec les groupes alkyle, alkoxy, aryle et siloxy tels que définis dans le cadre de la présente invention. Lorsque l'un au moins des X représente plusieurs groupes siloxy, lesdits groupes siloxy peuvent se répéter plusieurs fois de manière à conduire à des organosilanes polymériques de formule générale

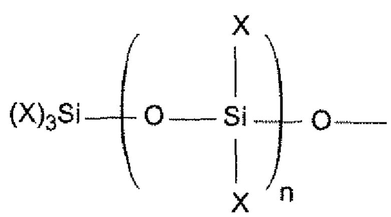

dans laquelle X est tel que défini ci-dessus et n est un nombre entier compris entre 1 et 20000, avantageusement entre 1 et 5000, plus avantageusement entre 1 et 1000. A ce titre on peut citer, par exemple, le polydiméthyl siloxane (PDMS), le polyméthylhydroxysiloxane (PMHS) et le tétraméthyldisiloxane (TMDS).

Par groupe « siloxy », on entend un groupe silylé, tel que défini ci-dessus, lié par un atome d'oxygène (-O-$Si(X)_3$) avec X tel que défini ci-dessus.

Au sens de l'invention, par « hétérocycle silylé », on entend un substituant mono- ou polycyclique, comportant de 5 à 15 membres, saturé ou instauré, contenant au moins un atome de silicium et éventuellement au moins un autre hétéroatome choisi parmi l'azote, l'oxygène et le soufre. Ledit hétérocycle silylé peut être éventuellement substitué par un ou plusieurs groupes hydroxyle ; un ou plusieurs groupes alkyle, un ou plusieurs groupes alkoxy ; un ou plusieurs atomes d'halogène choisis parmi les atomes de fluor, chlore, brome et iode ; un ou plusieurs groupes aryle, avec les groupes alkyle, alkoxy et aryle tels que définis dans le cadre de la présente invention. Parmi les hétérocycles silylés, on peut citer par exemple, le 1-silacyclo-3-pentène ou le 1-méthyl-1,1-dihydrido-2,3,4,5-tétraphényl-1-silacyclopentadiène, suivant les formules de la Figure 5.

On peut encore citer, par exemple, le méthyl siloxane, le 1-phényl-1-silacyclohexane, le 1-sila-bicyclo[2.2.1]heptane, le 1-méthyl-1-silacyclopentane, le 9,9-dihydro-5-silafluorène répondant aux formules de la Figure 6.

Par polyol on entend : un composé organique caractérisé par la présence d'un certain nombre de groupements hydroxyle (-OH). Dans le cadre de cette invention un composé polyol contient au moins un groupement hydroxyle. Pour ce faire, on entend par polyol un composé de formule $Z\text{-}(OH)_n$, dans lequel n est supérieur ou égale à 1, et Z est choisi parmi un ou plusieurs groupes alkyle, un ou plusieurs groupes alkoxy, un ou plusieurs groupes siloxy, un ou plusieurs groupes aryle, un ou plusieurs groupes hétéroaryle avec les groupes alkyle, alkoxy et aryle tels que définis dans le cadre de la présente invention.

Par groupe « amino », on entend un groupe de formule $-NR^4R^5$, dans laquelle ; ▪ $R^4$ et $R^5$ représentent, indépendamment l'un de l'autre, un atome d'hydrogène, un groupe alkyle, un groupe alcényle, un groupe alcynyle, un groupe aryle, un groupe hétéroaryle, un hétérocycle, un groupe silylé, un groupe siloxy, avec les groupes alkyle, alcényle, alcynyle, aryle, hétéroaryle, hétérocycle, silylé, siloxy, tels que définis dans le cadre de la présente invention ; ou

▪ $R^4$ et $R^5$, pris ensemble avec l'atome d'azote auquel ils sont liés, forment un hétérocycle éventuellement substitué par un ou plusieurs groupes hydroxyle ; un ou plusieurs groupes alkyle ; un ou plusieurs groupes alkoxy ; un ou plusieurs atomes d'halogène choisis parmi les atomes de fluor, chlore, brome et iode ; un ou plusieurs groupes nitro ($-NO_2$) ; un ou plusieurs groupes nitrile (-CN) ; un ou plusieurs groupes aryle ; avec les groupes alkyle, alkoxy et aryle tels que définis dans le cadre de la présente invention.

Par « bois » on entend un tissu végétal qui correspond au xylème secondaire chez les plantes. Le terme bois englobe tous les tissus secondaires formant les troncs, branches et racines des plantes ligneuses.

Par « lignine » on entend un biopolymère présent dans toutes les plantes et principalement dans les plantes vasculaires, les plantes ligneuses, les plantes herbacées et les algues. La lignine constitue un des principaux composants du bois. La lignine est un polyol riche en groupes aryles tels que définis ci-dessus. Elle est issue d'un tissu végétal notamment des feuilles, des tiges herbacées et des tiges ligneuses. En fonction de son procédé d'extraction et de son origine, la lignine peut contenir d'autres groupes chimiques comme par exemple, des alcènes, des alcynes, des alcools primaires secondaires et tertiaires, des cétones, des acides carboxyliques, des acétals, des hémiacétals, des énols, des éthers, des esters, des alcools allyliques, des alcools homoallyliques, des nitriles, des imines, des amines primaires secondaires et tertiaires, des amides, des halogènes, des sulfures, des thiols, des sulfonates, des sulfones, des sulfates, des sulfoxydes.

Dans le procédé de l'invention, le choix de l'espèce végétale dont sera extraite la lignine ainsi que son procédé d'extraction jouent un rôle important, notamment, sur la nature de la molécule contenant 1 à 2 cycles aromatiques que l'on cherche à obtenir et donc la sélectivité du procédé de l'invention, sur le rendement du procédé de l'invention, sur le degré de pureté dudit composé aromatique et sur la productivité de l'étape de dépolymérisation. Plus le pourcentage de résidu susceptible de donner la molécule désirée augmente dans l'espèce, plus la productivité du processus augmente.

La sélection de l'espèce végétale se réalise en tenant compte des paramètres décrits ci-après.

a) L'espèce végétale sélectionnée doit contenir avantageusement un pourcentage relativement élevé, c'est-à-dire au moins 10 % en masse de la lignine par rapport à la masse totale de l'échantillon de l'espèce végétale sélectionnée, ceci afin d'augmenter le rendement global de la molécule finale contenant 1 à 2 cycles aromatiques par rapport au matériau de départ, comme par exemple le bois.

b) L'espèce végétale doit être sélectionnée de sorte à avoir au moins 50% de résidu G, H ou S par rapport au nombre total de résidus présents dans la lignine utilisée. Il est à noter que le pourcentage des résidus présents dans la lignine peut être déterminé par des techniques connues par l'homme du métier, par exemple, la pyrolyse, la RMN, etc. Ce paramètre joue un rôle important dans la sélectivité par rapport à la molécule contenant 1 à 2 cycles aromatiques qui sera obtenue ainsi que dans l'augmentation du rendement de ladite molécule contenant 1 à 2 cycles aromatiques. Comme déjà indiqué, la lignine contient les résidus p-hydroxyphényle (H), guaiacyle (G), et

syringyle (S). Toutefois, la complexité ainsi que la structure de la lignine dépend largement de son origine. Or, en se basant sur la classification des lignines, on peut identifier quatre types : G, GS, HGS et HG.
Le type G peut être distingué des autres étant donné que les espèces contenants ce type de lignine sont exclusivement faites de résidus de type G. Ce type de lignine est en général issu des gymnospermes (softwood ou bois mou) et plus précisément de la division des conifères (Pinophyta) qui compte 600-650 espèces végétale.
Pour les autres types de résidus, les espèces naturelles contiennent toujours des mélanges GS ou HG, et les lignines issues de ces espèces sont caractérisées par le rapport S/G ou H/G. Toutefois il existe des espèces qui peuvent être riche en résidu S ou H. A titre d'exemple, on peut citer l'eucalyptus commun (*Eucalyptus globulus*) qui a un rapport S/G de 6.
Il existe également des espèces végétales génétiquement modifiées de sorte à avoir préférentiellement un seul résidu très majoritaire. Par exemple Mansfield est passé d'un ratio S/G = 2 à un ratio S/G = 12 pour le peuplier (J. J. Stewart, T. Akiyama, C. Chapple, J. Ralph, and S. D. Mansfield, Plant Physiol. 2009, 150, pages 621-635). Il est à noter que même si ces rapports entre les résidus ne sont pas respectés, par exemple, dans le cas où un mélange de résidu est contenu dans l'espèce, la productivité diminuera mais les produits finaux pourront être séparés par une méthode connue par l'homme du métier par exemple la distillation fractionnée ou la chromatographie sur colonne.
c) L'espèce doit de même être avantageusement sélectionnée de façon à maximiser, c'est-à-dire avoir au moins 30% de liaisons clivables par rapport au nombre total de liaisons présentes entre les motifs monomères dans la lignine. Au sens de l'invention, on entend par liaisons clivables, les liaisons carbone $sp^3$-oxygène qui pourront être clivées par l'étape de dépolymérisation et qui conduisent à la coupure de toutes les connections entre deux entités monomères successifs présents dans la lignine. Dans le cadre de la présente invention, le clivage sélectif des liaisons carbone $sp^3$-oxygène vise les liaisons de types β-O-4 et α-O-4. Dans le procédé de l'invention, la lignine mise en œuvre peut être, par exemple, une lignine contenant au moins 30% de liaison du type β-O-4 et/ou au moins 3% de liaisons α-O-4. Dans le cas de la lignine de bois, les liaisons de types β-O-4 et α-O-A constituent entre 40 et 60 % des liaisons présentes. Les pourcentages indiqués correspondent au pourcentage d'un type de liaison par rapport au nombre total de liaisons présentes entre les motifs monomères dans la lignine. Ce pourcentage peut être déterminé par la RMN, la pyrolyse, par exemple.
La lignine comporte également des liaisons modifiables mais non clivables comme les liaisons β-5, β-1, β-β, L'étape de dépolymérisation de la lignine dans le procédé de l'invention modifie ces liaisons mais un lien est toujours conservé entre les monomères aromatiques de la lignine.
Enfin, la troisième catégorie de liaison regroupe les liaisons non clivables et non modifiables comme les liaisons 4-O-5, 5-5. Dans le cadre de la présente invention, ces liaisons sont inertes et restent intactes dans les conditions opératoires appliquées. Il est donc important de sélectionner une lignine avec le plus de liaisons clivables possible (au moins 30% de liaisons clivables par rapport au nombre total de liaisons présentes entre les motifs monomères dans la lignine) pour pouvoir réaliser une dépolymérisation réussie de la lignine en des fragments similaires aux monolignols de départ comme montrés dans la Figure 1.

L'espèce végétale est de préférence sélectionnée de façon à augmenter les liaisons clivables β-O-4 et α-O-4 et de façon à avoir un type de résidu majoritaire H, G ou S dans la lignine.
Ainsi, l'espèce végétale est de préférence sélectionnée de façon à avoir :

- au moins 10 % en masse de la lignine par rapport à la masse totale de l'échantillon de l'espèce végétale sélectionnée ;
- au moins 30 % de liaisons clivables par rapport au nombre total de liaisons présentes entre les motifs monomères dans la lignine ; et
- au moins 50 % de résidu G, H ou S du nombre total de résidus présents dans la lignine utilisée.

Les espèces végétales peuvent être choisies, par exemple, parmi

- les cèdres, les pins, les épicéas, les sapins pour viser la formation d'une molécule contenant 1 à 2 cycles aromatiques ayant une structure dérivée du motif G ; ou
- les peupliers, les chênes, les eucalyptus dans le but de générer une molécule contenant 1 à 2 cycles aromatiques ayant une structure dérivée du motif S.

Une fois l'espèce végétale sélectionnée, elle doit être traitée de manière à extraire la lignine Au sens de l'invention, le procédé d'extraction de la lignine désigne toute technique physique et chimique permettant d'extraire, d'isoler, de séparer, de préparer la lignine. A titre d'exemple, on peut citer les procédés organosolv qui correspondent aux procédés utilisant un ou plusieurs solvants organiques pour extraire la lignine (*i.e.* les procédés : Acetocell, Alcell, Acetosolv, ASAM, Organocell, Milox, Formacell, Batelle/Geneva phénol), le procédé Steam-explosion, le procédé Klason, le procédé soda-AQ (produisant la lignine soda ou la lignine alkaline).

Les procédés organosolv sont décrits par les références suivantes :

a) Alcell : J. H. Lora, W. G. Classer, J Polym Environ, 2002, 10, 39-48 ;
b) Acetocell : Bojan Jankovic, Bioresource Technol., 2011, 102, 9763-9771 ;
c) Acetosolv : J. C. Parajo, J. L. Alonso, D. Vazquez, Bioresource Technology, 1993, 46, 233-240 ;
d) ASAM : I. Miranda, H. Pereira, Holzforschung, 2002, 56, 85-90 ;
e) Batelle/Geneva phénol : A. Johansson, O. Aaltonen, P. Ylinen, Biomass 1987, 13, 45-65 ;
f) Formacell : X.F. Sun, R.C. Sun, P. Fowler, M.S, Baird, Carbohydr. Polym., 2004, 55, 379-391;
g) Milox: P. Ligero, A. Vega, J.J. Villaverde, Bioresource Technol.,2010, 101, 3188-3193;
h) Organocell : A. Lindner, G. Wegener, J. Wood Chem, Technol. 1988, 8, 323 -340.

Dans le cas du bois, les procédés d'extraction précités permettent la séparation des trois principaux constituants du bois : la cellulose, l'hémicellulose et la lignine. Les procédés précités sont principalement basés sur des transformations chimiques ou thermochimiques, ce qui conduit à la modification de la structure de la lignine extraite. Cela signifie qu'un bois issu d'une même espèce peut engendrer différentes structures de lignine et ceci en fonction du procédé d'extraction employé.

Le procédé d'extraction de la lignine est choisi de sorte à modifier le moins possible la structure de la lignine initiale présente dans l'espèce.

Ainsi la lignine issue du procédé d'extraction garde les mêmes types de fonctionnalités et les mêmes proportions des liaisons que ceux présents dans la lignine de départ. Cela contribue à l'augmentation du rendement global du procédé de l'invention ainsi que la sélectivité par rapport à une molécule contenant 1 à 2 cycles aromatiques donnée. Le procédé d'extraction dans la présente invention est, de préférence, choisi parmi les procédés de types organosolv qui permettent d'obtenir des lignines dont la structure est très proche de celle de la lignine initiale présente dans l'espèce.

Dans le cadre de l'invention, le procédé d'extraction de la lignine peut englober également les procédés de traitement de la lignine dans le but d'introduire des fonctionnalisations chimiques, de changer les propriétés physiques et/ou de modifier la masse molaire moyenne de la lignine. La lignine est un polymère formé par une distribution de fragments polymériques ayant différentes masses molaires. La masse molaire moyenne de la lignine correspond donc à la moyenne des masses de ces fragments polymériques ; elle peut être calculée par rapport à la masse des fragments ou par rapport à leurs nombres. Ces procédés de traitements peuvent améliorer le rendement et la sélectivité du procédé de l'invention. Ainsi, à l'issue des procédés d'extraction précités, la lignine peut être traitée afin de modifier le ratio des résidus H, G et S qui la constituent. Cette modification peut aussi, dans certains cas, conduire à un enrichissement en un résidu donné et/ou en un type de liaison donné, par la suite, à augmenter la sélectivité ainsi que la productivité et réduire les étapes de purification conduisant au composé aromatique monocyclique final. Il est essentiel que la lignine obtenue soit exempte de soufre, c'est-à-dire qu'elle contienne un taux de soufre inférieur à 1,5 % en masse, par rapport à la masse totale de la lignine. En effet, les inventeurs ont observé, de manière tout à fait inattendue, que lorsque la lignine contient un taux de soufre égal ou supérieur à 1,5 % en masse, par rapport à la masse totale de la lignine, la dépolymérisation de la lignine par le procédé de l'invention n'a pas lieu ou elle est partielle. Lorsque la dépolymérisation est partielle, elle conduit à des molécules de masse molaire moyenne en poids supérieure à 1500 g/mol pour les molécules sous forme silylée ou de masse molaire moyenne en poids égale ou inférieure à 450g/mol, de préférence égale ou inférieure à 400g/mol (c'est-à-dire des molécules dont toutes les liaisons O-Si ont été clivées par exemple par une hydrolyse). Le taux de soufre de la lignine mise en œuvre dans le procédé de l'invention est donc avantageusement, égal ou supérieur à zéro et reste inférieur à 1,5 % en masse, par rapport à la masse totale de la lignine, comme défini ci-dessous :

$0 \leq$ taux de soufre de la lignine $< 1,5$ % en masse,

par rapport à la masse totale de la lignine.

Le taux de soufre peut être déterminé par les techniques physiques et chimiques connues de l'homme du métier, comme par exemple l'analyse élémentaire, le dosage par chromatographie ionique, par spectrophotométrie infrarouge, par oxydation du soufre en $SO_2$ puis dosage de ce dernier par les techniques connues de l'homme du métier, comme par exemple, le dosage acidimétrique, le dosage iodométrique, le dosage complexométrique.

Selon une variante préférée de l'invention, dans le composé silane de formule (I), $R^1$, $R^2$ et $R^3$ représentent, indépendamment l'un de l'autre, un atome d'hydrogène, un groupe alkyle, un groupe alkoxy, un groupe amino, un groupe aryle, un groupe silylé de formule [-Si(X)$_3$] avec X tel que défini précédemment avec l'un au moins des X représentant plusieurs groupes siloxy, lesdits groupes siloxy peuvent se répéter plusieurs fois de manière à conduire à des organosilanes polymériques de formule générale

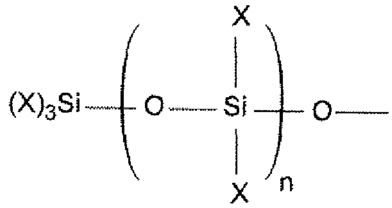

dans laquelle n est un nombre entier compris entre 1 et 20000, avantageusement entre 1 et 5000, plus avantageusement entre 1 et 1000,
lesdits groupes alkyle, alkoxy et aryle étant éventuellement substitués.

Plus préférentiellement, dans le composé silane de formule (I), $R^1$, $R^2$ et $R^3$ représentent indépendamment l'un de l'autre, un atome d'hydrogène ; un groupe alkyle choisi parmi les groupes méthyle, éthyle, propyle, butyle, pentyle, hexyle, heptyle et leurs isomères ramifiés ; un groupe alkoxy dont le groupe alkyle est choisi parmi les groupes méthyle, éthyle, propyle, butyle, pentyle, hexyle, heptyle et leurs isomères ramifiés ; un groupe aryle choisi parmi les groupes benzyle et phényle ; un groupe silylé tel que décrit ci-dessus choisi parmi le polydiméthyl siloxane (PDMS), le polyméthylhydroxysiloxane (PMHS) et le tétraméthyldisiloxane (TMDS).

Par catalyseur, au sens de l'invention, on entend tout composé capable de modifier, notamment en augmentant, la vitesse de la réaction chimique à laquelle il participe, et qui est régénéré à la fin de la réaction. Cette définition englobe à la fois les catalyseurs, c'est-à-dire les composés qui exercent leur activité catalytique sans avoir besoin de subir une quelconque modification ou conversion, et les composés (appelés également pré-catalyseurs) qui sont introduits dans le milieu réactionnel et qui y sont convertis en un catalyseur.

Il est en particulier nécessaire que le catalyseur soit choisi en tenant compte notamment de son encombrement stérique, de son aptitude à activer le silane et de sa solubilité dans le milieu réactionnel.

Dans le procédé de l'invention, le catalyseur peut être un catalyseur organique choisi parmi :

- les carbocations de formule $(X^1)_3C^+$ avec $X^1$ représentant un atome d'hydrogène, un groupe alkyle, un groupe aryle, un groupe alkoxy, un groupe silyle, un groupe siloxy et un atome d'halogène, tels que définis ci-dessus, lesdits carbocations étant choisis parmi le cation trityle ($(C_6H_5)_3C^+$), le tropilium $(C_7H_7)^+$ le cation benzylique ($C_6H_5CH_2^+$), le cation allylique ($CH_3$-$CH^+$-$CH{=}CH_2$), le méthylium ($CH_3^+$), le cyclopropylium ($C_3H_5^+$), le carbocation cyclopropylique de formule C-$_3H_5$-$C^+R^1R^2$ avec $R^1$ et $R^2$ comme définis ci-dessus ledit carbocation étant choisi parmi le carbocation diméthyle cyclopropylique et le carbocation dicyclopropylique, l'acylium ($R^1$-C=O)$^+$ avec $R^1$ comme défini ci-dessus et choisi parmi le méthyle, le propyle et le benzyle, le cation benzénium $(C_6H^5)^+$, le cation norbornyle $(C_7H_{11})^+$ ;
- les oxoniums choisis parmi $(CH_3)_3O^+BF_4^-$ (sel de Meerwein) et $(CH_3CH_2)_3O^+BF_4^-$ ;
- un ion silylium $(R^1)_3Si^+$ avec $R^1$ tel que défini précédemment, par exemple, choisi parmi $Et_3Si^+$ et $Me_3Si^+$ ;
- les cations disilyles, de préférence les cations disilyles ayant un hydrure pontant choisis parmi les formules indiquées ci-dessous

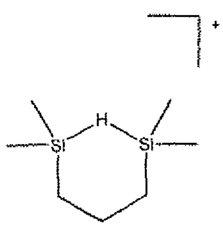

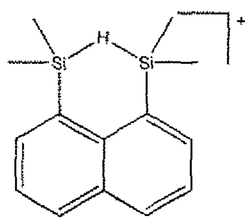

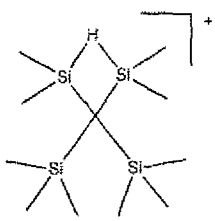

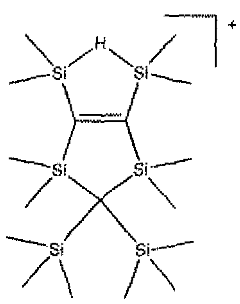

lesdits cations disilyles pouvant être synthétisés par l'homme du métier comme décrit par R. Panisch, M. Boite, and T. Muller, J. Am. Chem. Soc. 2006, 128, pages 9676-9682).

Les carbocations cités ci-dessus sont commerciaux ou peuvent être facilement synthétisés par l'homme du métier par différents procédés de synthèse, par exemple : le procédé de bassin de cation (cation pool), le procédé redox interne, le procédé utilisant un groupement partant, les procédés utilisant des acides de Lewis ou de Bronsted. Ces procédés sont décrits dans les références suivantes : R. R. Naredla et D. A. Klumpp, Chem. Rev. 2013, 113, pages 6905-6948 ; M. Saunders. et H. A. Jimenez-Vazquez, Chem. Rev. 1991, 91, pages 375-397.

Il est à noter que le contre ion anionique de l'ion silylium, des carbocations et des cations disilyles précités est, de préférence, un halogénure choisi parmi $F^-$, $Cl^-$, $Br^-$ et $I^-$, ou un anion choisi parmi $BF_4^-$, $SbF_6^-$, $B(C_6F_5)_4^-$, $B(C_6H_5)_4^-$, $TfO^-$ ou $CF_3SO_3^-$, $PF_6^-$.

Dans le procédé de l'invention, le catalyseur peut être également organométallique. A ce titre on peut citer les complexes d'iridium ($[(PX^2CX^2P)Ir(R^7)(S)]^+Y^-$), de formule (III)

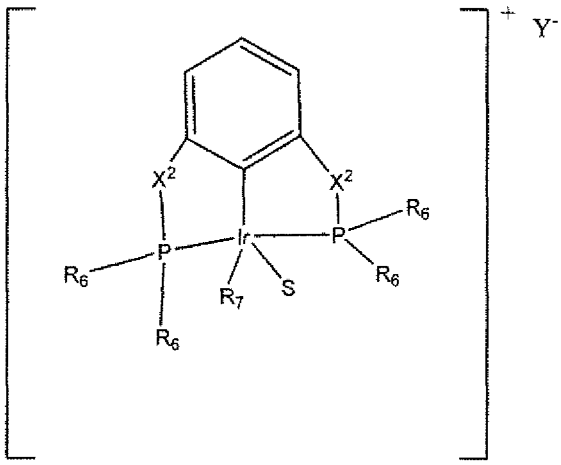

(III)

dans laquelle

- $R^6$ représente un groupe alkyle ou aryle tel que défini précédemment, et de préférence un groupe iso-butyle ;
- $R^7$ représente un atome d'hydrogène ou un groupe alkyle tel que défini précédemment, et de préférence un atome d'hydrogène ; et
- $X^2$ représente un groupe $-CH_2-$ ou un atome d'oxygène, et de préférence un atome d'oxygène ;
- Y représente un contre ion choisi parmi $B(C_6F_5)_4$ et $B(C_6H_5)_4$, et de préférence $B(C_6F_5)_4$ ;
- S représente une molécule de solvant, coordonnée au complexe, choisi parmi le diméthylesulfoxyde (DMSO), l'acétonitrile ($CH_3CN$) et l'acétone ($CH_3COCH_3$), et de préférence l'acétone.

Selon un mode de réalisation préféré de l'invention, le catalyseur d'iridium est $[(POCOP)Ir(H)(acétone)^+B(C_6F_5)_4^-$ avec (POCOP) représentant le 2,6-bis(di-tert-butylphosphinito)phényle.Ce catalyseur peut être préparé selon les procédés décrits par I. Gottker-Schnetmann, P. White, et M. Brookhart, J. Am. Chem. Soc. 2004, 126, pages 1804-1811 ; et par J. Yang et M. Brookhart, J. Am. Chem. Soc. 2007, 129, pages 12656-12657.

Dans le procédé de l'invention, le catalyseur peut être également organométallique. A ce titre on peut citer les complexes de ruthénium de formule (IV)

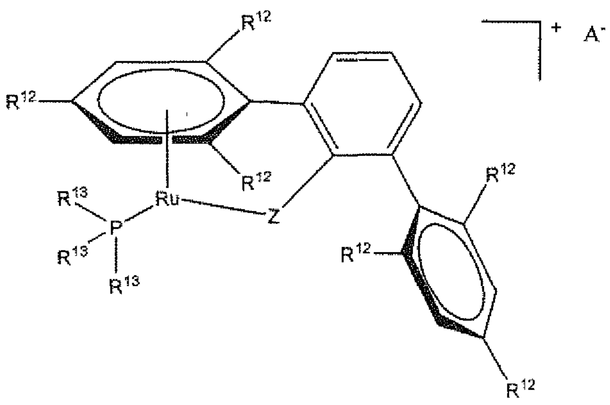

(V)

dans laquelle

- $R^{12}$ représente un atome d'hydrogène ou un groupe alkyle tel que défini précédemment, $R^{12}$ étant de préférence un groupe méthyle ;
- $R^{13}$ représente un aryle ou un groupe alkyle tel que défini précédemment, lesdits groupes aryle et alkyle étant éventuellement substitués, $R^{13}$ étant de préférence $p$-$FC_6H_4$ ;
- Z représente un groupe $-CH_2-$, un atome d'oxygène ou un atome de soufre, Z étant de préférence un atome de soufre ; et
- $A^-$ représente un contre ion choisi parmi $B(C_6F_5)_4^-$ et $[CHB_{11}H_5Cl_6]^-$, $A^-$ étant de préférence $B(C_6F_5)_4^-$.

Ce type de catalyseur peut être préparé selon les procédés décrits par T. Stahl , H. F. T. Klare, and M. Oestreich, J. Am. Chem. Soc., 2013, 135, pages 1248-1251.

Le catalyseur peut également être de type acide de Lewis choisi parmi les catalyseurs organométalliques et métalliques :

- les composés de bore de formule $B(X^3)_3$ avec $X^3$ représentant un atome d'hydrogène, un groupe alkyle, un groupe aryle, un groupe alkoxy tels que définis précédemment, lesdits composés de bore étant choisis parmi $BF_3$, $BF_3(Et_2O)$, $BCl_3$, $BBr_3$, le triphényle hydroborane, le tricyclohexyle hydroborane, $B(C_6F_5)_3$, le B-méthoxy-9-borabicyclo[3.3.1]nonane (B-méthoxy-9-BBN), le B-benzyl-9-borabicyclo[3.3.1]nonane (B-benzyl-9-BBN) ;
- les composés boréniums $R^1R^2B^+$ avec $R^1$ et $R^2$ tels que définis précédemment, lesdits composés boréniums étant par exemple Me-TBD-BBN$^+$, les dérivés borenium ferrocène répondant à la formule

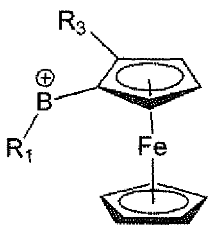

borenium ferrocène

dans laquelle $R^1$ et $R^3$ tels que définis précédemment, par exemple, $R^1$ est un groupe phényle et $R^3$ est le 3,5-diméthylpyridyle ;
- les composés de l'aluminium choisis parmi $AlCl_3$, $AlBr_3$, l'isopropoxyde d'aluminium $Al(O\text{-}i\text{-}Pr)_3$, l'éthanoate d'aluminium $(Al(C_2H_3O_2))$, le sel de Krossing $[Ag(CH_2Cl_2)]\{Al[OC(CF_3)_3]_4\}$, le $Li\{Al[OC(CF_3)_3]_4\}$, les composés cationiques d'aluminium de formule $(X^4)_2Al^+$ avec $X^4$ étant un atome d'halogène, un groupe alkoxy, un groupe alkyle tels que définis précédemment comme, par exemple, $Et_2Al^+$ ;
- les composés d'indium choisis parmi $InCl_3$, $In(OTf)_3$ ;
- les composés de fer choisis parmi $FeCl_3$, $Fe(OTf)_3$ ;
- les composés de l'étain choisis parmi $SnCl_4$, $Sn(OTf)_2$ ;
- les composés du phosphore tels que $PCl_3$, $PCl_5$, $POCl_3$ ;

- les composés trifluorométhanesulfonates ou triflates ($CF_3SO_3^-$) de métaux de transitions et des lanthanides choisis parmi le triflate de scandium, le triflate de ytterbium, le triflate d'yttrium, le triflate de cérium, le triflate de samarium, le triflate de niodinium.

Dans le cadre de la présente invention OTf représente l'ion triflate ou trifluorométhanesulfonate de formule $CF_3SO_3^-$: les termes triflate ou trifluorométhanesulfonate, OTf ou $CF_3SO_3^-$ peuvent donc être utilisés indifféremment pour désigner la même entité.

La préparation des dérivés borenium ferrocène est décrite par J. Chen, R. A. Lalancettea et F. Jäkle, Chem. Commun., 2013, 49, pages 4893-4895) ; la préparation des sels de Krossing est décrite par I. Krossing, Chem.-Eur. J., 2001, 7, page 490 ; et la préparation de $Et_2Al^+$ est décrite par M. Khandelwal et R. J. Wehmschulte, Angew. Chem. Int. Ed. 2012, 51, pages 7323 -7326.

Selon une variante préférée de l'invention, le catalyseur est un catalyseur organométallique choisi parmi $BF_3$ ; $InCl_3$ ; le triphénylcarbénium tetrakis(perfluorophenyl)borate $[(Ph)_3C^+B(C_6F_5)_4^-,B(C_6F_5)_3]$.

Certaines des abréviations utilisées dans le cadre de l'invention sont représentées dans la Figure 7.

Les catalyseurs peuvent, le cas échéant, être immobilisés sur des supports hétérogènes afin d'assurer une séparation facile dudit catalyseur et/ou son recyclage. Lesdits supports hétérogènes peuvent être choisis parmi les supports à base de gel de silice et de polymères plastiques comme, par exemple, le polystyrène ; les supports carbonés choisis parmi les nanotubes de carbone ; le carbure de silice ; l'alumine ; et le chlorure de magnésium ($MgCl_2$).

Dans le procédé selon l'invention, la réaction peut se produire sous une pression d'un ou d'un mélange de gaz inerte(s) choisi(s) parmi l'azote et l'argon, ou des gaz générés par le procédé notamment du méthane et d'hydrogène. La pression peut être comprise entre 0,2 et 50 bars, de préférence entre 0,2 et 30 bars, plus préférentiellement entre 1 et 20 bars, bornes incluses.

La température de la réaction peut être comprise entre 0 et 150°C, de préférence entre 0 et 125°C, plus préférentiellement entre 25 et 70°C, bornes incluses.

La durée de la réaction dépend du taux de conversion du composé silane de formule (I), de la nature de la lignine ainsi que du taux de silylation souhaité.

La réaction peut être effectuée pendant une durée de 1 minute à 200 heures, avantageusement de 1 minute à 48 heures, de préférence de 10 minutes à 48 heures, bornes incluses.

Le procédé de l'invention, en particulier la réaction entre les différents réactifs, peut avoir lieu dans un ou un mélange d'au moins deux solvant(s) choisi(s) parmi :

- les éthers silylés, de préférence, choisis parmi le 1,1,1,3,3,3-hexaméthyldisiloxane ($(Me_3Si)_2O$), le 1,1,1,3,3,3-hexaéthyldisiloxane ($(Et_3Si)_2O$).
- les hydrocarbures, de préférence, choisis parmi le benzène, le toluène, le pentane et l'hexane ;
- les sulfoxydes, de préférence, choisis parmi le diméthylesulfoxyde (DMSO) ;
- les halogénures d'alkyle, de préférence, choisis parmi le chloroforme, le chlorure de méthylène, le chlorobenzène, le dichlorobenzène.

Les silanes de formule (I) et les catalyseurs utilisés dans le procédé de l'invention sont, en général, des composés commerciaux ou peuvent être préparés par les procédés connus de l'homme du métier.

Le rapport massique entre le composé silane de formule (I) et la lignine dépend du type de lignines employées et du type de molécules finales souhaitées (obtention des éthers silylés du type IIb, IId, IIf comme représentés dans les exemples ou obtention des éthers silylés du type IIa, IIc, IIe comme représentés dans les exemples). Les composés IIa-IIf sont donc des éthers silylés qui peuvent être déprotégés pour conduire aux alcools correspondants de formule (IV) par hydrolyse. L'hydrolyse des éthers silylés peut être réalisée par les techniques d'hydrolyse chimique (condition acide ou basique) connues de l'homme du métier. Une hydrolyse enzymatique peut également être mise en œuvre. Des exemples d'hydrolyse sont fournis dans les exemples de réalisation du procédé de l'invention.

Les éthers silylés contant une chaîne propyle substitué de formule IIb, IId, IIf peuvent également donner d'autres éthers silylés contenant une chaine propyle non substitué du type IIa, IIc, IIe par le même procédé que celui utilisé pour la dépolymérisation de la lignine. Comme le procédé de dépolymérisation réduit les liaisons carbone sp3-oxygène, les liaisons silylées (-C-O-Si-) peuvent être facilement réduites en alcane (-C-H). L'obtention des éthers silylés contenant une chaîne propyle non substitué dépendra du nombre d'équivalents de composé silane de formule (I) ajouté.

Ainsi, dans le cadre de la présente invention, le rapport massique entre le composé silane de formule (I) et la lignine peut être compris entre 0,5 et 6, de préférence entre 1 et 4, bornes incluses.

La quantité de catalyseur utilisé dans le procédé de l'invention est de 0,001 à 1 équivalent en masse, de préférence de 0,001 à 0,9 équivalent en masse, plus préférentiellement de 0,01 à 0,9 équivalent en masse, encore plus préférentiellement de 0,01 à 0,5 équivalent en masse, bornes incluses, par rapport à la masse initiale de la lignine.

Comme déjà indiqué, la dépolymérisation de la lignine conduit à l'obtention de molécules aromatiques contenant

1 à 2 cycles aromatiques de masse molaire moyenne en poids inférieure à 1500 g/mol pour les molécules sous forme silylée ou de masse molaire moyenne en poids égale ou inférieure à 450g/mol, de préférence égale ou inférieure à 400g/mol (soit un degré de polymérisation inférieur à 3 unités monomères, de préférence entre 1 et 2 unités monomères). La masse molaire moyenne en poids des composés aromatiques et le degré de polymérisation de la lignine peuvent être déterminés par les techniques habituelles utilisées dans ce domaine et connues de l'homme du métier comme, par exemple, la chromatographie d'exclusion stérique.

Après la dépolymérisation, les composés aromatiques résultants sont en général au moins partiellement sous forme silylée, en particulier sur les résidus phénoliques de la lignine. Toutefois, une simple hydrolyse dans des conditions bien connues de l'homme du métier, conduit aux composés aromatiques correspondant sous leurs formes non silylées.

Dans le cadre de la présente invention, par hydrolyse on entend un procédé de transformation des groupements siloxy présents dans des composés aromatiques silylés issus de dépolymérisation de la lignine, en groupements hydroxyles, par une réaction de désilylation. Cette transformation peut se réaliser dans des conditions acides ou basiques ou bien en présence d'ions fluorures, ces conditions étant bien connues de l'homme du métier. Dans le cadre de la présente invention, le procédé d'hydrolyse est, de préférence, choisi parmi : HCl ou $H_2SO_4$ 2 M dans le THF ; NaOH ou KOH 10 % dans un mélange eau/THF ; le fluorure de tétra-*n*-butylammonium (TBAF) 1 M dans le THF.

Une simple filtration peut permettre de récupérer le catalyseur éventuellement supporté et d'éliminer les éventuels sous-produits.

Ainsi, le procédé de l'invention permet à la lignine de devenir la principale source de composés aromatiques d'origine biologique pour l'industrie chimique. Des composés aromatiques de hautes valeurs ajoutées comme, par exemple, le benzène, le toluène, les xylènes (BTX), les coniférols substitués, le phénol, les polyols aromatiques, et les quinines peuvent ainsi être obtenus et utilisés dans la synthèse des résines phénol-formaldéhyde, des polymères polyoléfine-lignine, des polymères polyester-lignine, des polyuréthanes, des bio-plastiques, des résines époxydes.

Les composés aromatiques obtenus par le procédé de l'invention peuvent donc être utilisés comme matières premières dans les secteurs de la construction, dans les parfums, dans l'industrie pétrochimique, alimentaire, électronique, de textile, aéronautique, pharmaceutique, cosmétique, agrochimique.

L'invention a donc pour objet l'utilisation du procédé de dépolymérisation de la lignine selon l'invention, dans la fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais.

L'invention a, également, pour objet un procédé de fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais, caractérisé en ce qu'il comprend une étape de dépolymérisation de la lignine par le procédé selon l'invention.

Outre la bonne productivité et la bonne sélectivité, le procédé de l'invention permet d'utiliser

- la lignine qui représente le plus grand réservoir de composés aromatiques d'origine biologique, et
- un agent réducteur doux (le silane de formule (I)) stable à l'air et peu coûteux et compatible avec la présence éventuelle de groupements fonctionnels sur la lignine.

Le procédé de l'invention permet à la lignine de devenir la principale source de composés aromatiques d'origine biologique pour l'industrie chimique.

D'autres avantages et caractéristiques de la présente invention apparaîtront à la lecture des exemples ci-dessous donnés à titre illustratif et non limitatif.

## EXEMPLES

Le procédé de dépolymérisation de la lignine par clivage sélectif de la liaison carbone $sp^3$-oxygène des alkylaryle éthers présents dans la lignine est effectué en présence d'un catalyseur, en faisant réagir une lignine avec un taux de soufre inférieur à 1,5% en masse de lignine, avec un composé silane de formule (I) le protocole expérimental suivant.

Les réactifs utilisés, notamment le composé silane de formule (I) et le catalyseur sont des produits commerciaux.

### Protocole expérimental général de dépolymérisation de la lignine

1. Sous atmosphère inerte d'argon ou d'azote, le composé silane de formule (I), le catalyseur (de 1 à 0,001 équivalents massiques calculés par rapport à la masse initiale de la lignine ajoutée) et la moitié de la quantité de solvant sont mis sous agitation dans un récipient en verre de volume adapté. La concentration en silane dans le mélange

réactionnel varie de 1,0-6,0 mol.$L^{-1}$ (concentration calculée sur la base de la moitié du volume final de solvant introduit).

2. D'autre part, dans un tube de Schlenk, la lignine organosolv (10 - 40 % d'équivalent en masse de silane ajouté), préalablement séchée pendant la nuit à l'aide d'une rampe à vide, est placée sous agitation avec la moitié restante de solvant.

3. La solution contenant le catalyseur et le composé silane de formule (I) est ajoutée lentement (temps d'ajout 15 minutes-1 heure) à l'aide d'une seringue et sous agitation, au tube de Schlenk. Ce dernier est laissé ouvert afin d'évacuer les gaz produits par la réaction.

4. Après la fin de l'ajout de la solution et l'arrêt du dégagement gazeux, le tube de Schlenk est fermé et est laissé sous agitation. La lignine de départ est alors soluble presque totalement. Le suivi de réaction est réalisé par GC-MS.

5. Une fois la réaction terminée (temps de réaction de 1 à 72 heures), le solvant ainsi que les composés volatils sont évaporés à l'aide d'une rampe à vide ($10^{-2}$ mbar). Le liquide visqueux obtenu est purifié à l'aide d'une chromatographie sur gel de silice en utilisant un gradient d'élution de 100 : 0 jusqu'à 0 : 100 de pentane : $CH_2Cl_2$ pour les fractions apolaires, et un gradient d'élution de 100 : 0 à 0 : 100 de $CH_2Cl_2$ : AcOEt pour les fractions polaires. Dans le cas où une fraction est très polaire, l'élution peux se réaliser avec un mélange AcOEt : MeOH (50 : 50 à 0 : 100). Il est à noter qu'en fonction de l'application visée, l'étape de purification peut ou pas être omise.

6. Enfin, les différentes fractions issues de la colonne sont hydrolysées en milieu acide en utilisant HCl ou $H_2SO_4$ 2M dans le THF, ou en milieu basique en utilisant NaOH ou KOH 15 à 30 % en masse, ou finalement à l'aide d'un réactif fluoré type : HF-pyridine, TBAF, CsF, $NH_4F$ pour fournir le produit hydrolysé correspondant.

Un ensemble de résultats est présenté ci-dessous, donnant des exemples de dépolymérisation de la lignine organosolv.

Les catalyseurs testés sont le $B(C_6F_5)_3$ ainsi que le complexe d'iridium ($[(POCOP)Ir(H)(acetone)]^+B(C_6F_5)_4^-$) dont la synthèse est décrite par I. Gottker-Schnetmann, P. White, et M. Brookhart, J. Am. Chem. Soc. 2004, 126, pages 1804-1811 ; et par J. Yang et M. Brookhart, J. Am. Chem. Soc. 2007, 129, pages 12656-12657.

La lignine utilisée est issue de plusieurs procédés de type organosolv {a) Alcell : J. H. Lora, W. G. Glasser, J Polym Environ, 2002, 10, pages 39-48 ; b) Acetocell : Bojan Jankovic, Bioresource Technol., 2011, 102, pages 9763-9771 ; c) Acetosolv : J. C. Parajo, J. L. Alonso, D. Vazquez, Bioresource Technology, 1993, 46, pages 233-240 ; d) ASAM : I. Miranda, H. Pereira, Holzforschung, 2002, 56, pages 85-90 ; e) Batelle/Genevaphenol : A. Johansson, O. Aaltonen, P. Ylinen, Biomass 1987, 13, pages 45-65 ; f) Formacell : X.F. Sun, R.C. Sun, P. Fowler, M.S. Baird, Carbohydr. Polym., 2004, 55, pages 379-391 ; g) Milox: P. Ligero, A. Vega, J.J. Villaverde, Bioresource Technol., 2010, 101, pages 3188-3193 ; h) Organocell : A. Lindner, G. Wegener, J. Wood Chem. Technol. 1988, 8, pages 323-340} et en particulier le procédé AVIDEL (décrit par H. Q. Lam, Y. Le Bigot, M. Delmas, G. Avignon, Industrial Crops and Products, 2001, 14, pages 139-144) qui constitue une version optimisée du procédé Formacell.

Les types de bois desquelles les lignines sont issues, sont sélectionnés avec des proportions G/H/S différentes, de même un mélange de plusieurs types de bois a été employé, pour montrer la versatilité ainsi que la robustesse du procédé. Dans le contexte de l'invention, par «robustesse du procédé» on entend un procédé qui, dans des conditions opératoires très douces, permet la coupure des fonctions chimiques habituellement très dures à cliver.

## EXEMPLE 1 : Dépolymérisation de la lignine issue du Platane commun (*Platanus acerifolia*) (extraite par le procédé AVIDEL) en utilisant le triéthylsilane ($Et_3SiH$)

La dépolymérisation du Platane commun est réalisée en suivant le mode opératoire général de dépolymérisation décrit ci-dessus.

La dépolymérisation se réalise avec 4-5 mol.$L^{-1}$ $Et_3SiH$ comme silane (concentration calculée sur la base de la moitié du volume final de solvant introduit). La masse de lignine ajoutée correspond à 30 % de la masse de silane ajouté et le solvant utilisé est le dichlorométhane ($CH_2Cl_2$). La réaction se réalise en présence de 20-30 % en masse de catalyseur (masse calculée par rapport à la masse de lignine ajoutée). Le catalyseur utilisé est $B(C_6F_5)_3$.

La solution de silane et de catalyseur est ajoutée sur une période de 30 minutes dans le tube de Schlenk et la réaction est laissée sous agitation pendant 24 heures à 25°C. Après la fin de la réaction et l'évaporation du solvant et des volatiles, le liquide visqueux obtenu est purifié en utilisant les mêmes conditions que décrites précédemment. Ce liquide est constitué d'un mélange de produits de formules **IIa, IIb, IIc** et **IId** (identifiés par RMN et par GC-MS).

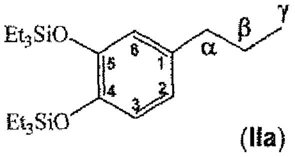
(IIa)

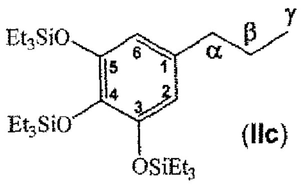
(IIc)

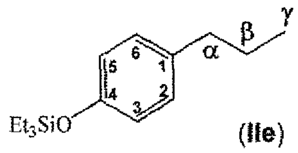
(IIe)

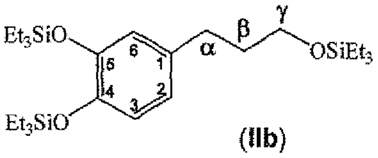
(IIb)

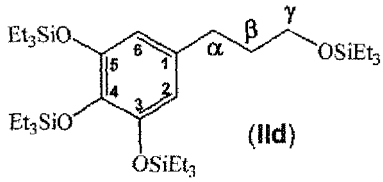
(IId)

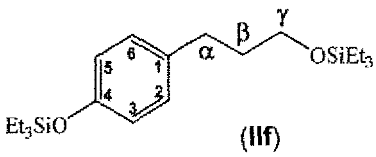
(IIf)

Le rapport molaire **IIa/IIb/IIc/IId** a été déterminé d'après l'analyse GC-MS (Appareil Shimadzu GCMS-QP2010 Ultra gas chromatograph mass spectrometer équippé avec une colonne capillaire de silice fisionnée : fused silica capillary column Supelco SLB™-ms (30 m x 0.25 mm x 0.25 μm) comme indiqué dans le Tableau 1. Enfin, les fractions issues de la purification ont été hydrolysées en milieu acide en utilisant une solution de 2 M de HCl dans le THF. Apres 16 heures d'agitation à température ambiante (20 ± 5°C), le solvant ainsi que les volatils sont évaporées, pour fournir les différents polyols correspondants.

**IIa** :

**$^1$H NMR** (200 MHz, $CDCl_3$, $Me_4Si$) δ (ppm) = 6.71 (1 H, d, $^3J$ = 8.1 Hz, Ar-H), 6.63 (1 H, s, Ar-H), 6.58 (1 H, d, $^3J$ = 8.1 Hz, Ar-H), 2.45 (2 H, t, $^3J$ = 7.8 Hz, Ar-$CH_2$), 1.57 (2 H, sex, $^3J$ = 7.8 Hz, $CH_2$-$CH_3$), 0.98 (18 H, t, $^3J$ = 7.9 Hz, $CH_3CH_2Si$), 0.90 (3 H, t, $^3J$ = 7.8 Hz, $CH_3CH_2Si$), 0.74 (12 H, q, $^3J$ = 7.9 Hz, $CH_3CH_2Si$).
**$^{13}$C NMR** (50 MHz, $CDCl_3$, $Me_4Si$): δ (ppm) =146.5, 144.7, 136.0, 121.3, 120.9, 120.2, 37.4, 24.7, 13.9, 6.9, 5.3, 5.2.
**HR-MS (APPI):** calculé (M +) ($C_{21}H_{40}O_2Si_2$), m/z 380.2566; trouvé (M +), m/z 380.2559. **Anal. Calculée.** for $C_{21}H_{40}O_2Si_2$ (masse molaire 380.72): C, 66.25; H, 10.59. **Trouvé:** C, 66.18; H, 10.46.
**MS:** IE (m/z): 380 (9); 351 (4); 207 (8) ; 117 (4) ; 116 (11) ; 115 (100) ; 88 (7) ; 87 (74) ; 59 (45); 58 (4).

**IIb :**

**$^1$H NMR** (200 MHz, $CDCl_3$, $Me_4Si$) δ (ppm) = 6.79 - 6.50 (3 H, m, Ar-H), 3.60 (2 H, t, $^3J$ = 6.6 Hz, $CH_2$-O), 2.54 (2 H, t, $^3J$ = 7.6 Hz, Ar-$CH_2$), 1.79 (2 H, quin, $^3J$ = 7.0 Hz, Ar-$CH_2$-$CH_2$), 1.05 - 0.88 (27 H, m, $CH_3CH_2Si$), 0.84 - 0.48 (18 H, m, $CH_3CH_2Si$).
**$^{13}$C NMR** (50 MHz, $CDCl_3$, $Me_4Si$): δ (ppm) =146.5, 144.8, 135.4, 121.3, 120.9, 120.3, 62.3, 34.7, 31.5, 6.9, 6.8, 5.2, 5.2, 4.6.
MS: IE (m/z): 87 (100), 115 (57), 59 (38), 89 (28), 207 (24), 32 (16), 235 (11), 88 (10), 337 (9), 511 (8), 116 (6), 86 (6).

**IIc :**

**$^1$H NMR** (200 MHz, $CDCl_3$, $Me_4Si$) δ (ppm) = 6.27 (2 H, s, Ar-H), 2.39 (2 H, t, $^3J$ = 7.5 Hz, Ar-$CH_2$), 1.69 - 1.45 (2 H, m, $CH_2$-$CH_3$), 1.1 - 0.84 (27 H, m, $CH_3CH_2Si$), 0.90 - 0.81 (3 H, m, $CH_3CH_2Si$), 0.83 - 0.65 (18 H, m, $CH_3CH_2Si$).
**$^{13}$C NMR** (50 MHz, $CDCl_3$, $Me_4Si$): δ (ppm) = 147.8, 146.5, 134.5, 113.6, 37.7, 24.6, 13.7, 7.0, 6.8, 5.4, 5.2.
**MS:** IE (m/z): 510 (8) ; 339 (4); 338 (10); 337 (31) ; 116 (7) ; 115 (60) ; 88 (10) ; 87 (100); 86 (4) ; 59 (49).

**IId** :

**$^1$H NMR** (200 MHz, $CDCl_3$, $Me_4Si$) δ (ppm) = 6.28 (2 H, s, Ar-H), 3.59 (2 H, t, $^3J$ = 6.7 Hz, $CH_2$-**O**), 2.48 (2 H, t, $^3J$ = 7.5 Hz, Ar-$CH_2$), 1.78 (2 H, quin, $^3J$ = 7.3 Hz, Ar-$CH_2$-$CH_2$), 1.13 - 0.85 (36 H, m, $CH_3CH_2Si$), 0.84 - 0.49 (24 H, m, $CH_3CH_2Si$).
**$^{13}$C NMR** (50 MHz, $CDCl_3$, $Me_4Si$): δ (ppm) =147.9, 136.6, 134.0, 113.6, 62.3, 34.6, 31.7, 6.9, 6.8, 5.4, 5.2, 4.5.
**MS:** IE (m/z): 87 (100), 115 (36), 59 (32), 89 (19), 641 (9), 88 (9), 467 (8), 365 (7), 337 (6), 642 (5), 640 (5), 116 (4).

**EXEMPLE 2 : Dépolymérisation de la lignine issue du pin (*Pinus pinea*) (extraite par le procédé AVIDEL) en utilisant le triéthylsilane ($Et_3SiH$)**

Le même mode opératoire employé pour la dépolymérisation de la lignine issue du Platane commun, est utilisé pour la dépolymérisation de la lignine issue du pin. Dans ce cas et après purification, le produit IIa est obtenu avec une très grande pureté (> 99,7%) avec un rendement massique de 10 à 20% par rapport à la masse de lignine employée (non optimisé). Ce produit a été caractérisé par GC-MS, RMN 13C, RMN 1H et par HR-MS. Enfin, l'hydrolyse des fractions issues de la purification se réalise en agitant à 25°C chaque fraction pendant 16h en présence d'une solution de 2 M de HCl dans le THF. Enfin, les polyols sont obtenus après l'évaporation du solvant et des composés volatils.

**EXEMPLE 3 : Dépolymérisation de la lignine issue du Peuplier d'Italie (*Populus nigra*) (extraite par le procédé AVIDEL) en utilisant le triéthylsilane ($Et_3SiH$)**

Le même mode opératoire employé pour la dépolymérisation de la lignine issue du Platane commun, est utilisé pour la dépolymérisation de la lignine issue du Peuplier d'Italie. De même, les produits obtenus dans les deux cas sont similaires. Parmi les produits les plus volatils, les produits e formules **IIa** et **IIc** sont identifiés par RMN et par GC-MS comme indiqué dans le Tableau 1.

**EXEMPLE 4 : Dépolymérisation de la lignine issue du Bouleau pendant (*Betula pendula*) (extraite par le procédé A VIDEL) en utilisant le triéthylsilane ($Et_3SiH$)**

Le même mode opératoire employé pour la dépolymérisation de la lignine issue du Platane commun, est utilisé pour la dépolymérisation de la lignine issue du Bouleau pendant. De même, les produits obtenus dans les deux cas sont similaires. Parmi les produits les plus volatils, les produits de formules **IIa** et **IIc** sont identifiés par RMN et par GC-MS comme indiqué dans le Tableau 1.

**EXEMPLE 5 : Dépolymérisation de la lignine issue du Hêtre Commun (*Fagus sylvatica*) (extraite par le procédé AVIDEL) en utilisant le triéthylsilane ($Et_3SiH$)**

Le même mode opératoire employé pour la dépolymérisation de la lignine issue du Platane commun, est utilisé pour la dépolymérisation de la lignine issue du Hêtre Commun. De même, les produits obtenus dans les deux cas sont similaires. Parmi les produits les plus volatils, les produits de formules **IIa** et **IIc** sont identifiés par RMN et par GC-MS comme indiqué dans le Tableau 1.

**EXEMPLE 6 : Dépolymérisation de la lignine issue de l'Eucalyptus (*Eucalyptus camaldulensis)* (extraite par le procédé AVIDEL) en utilisant le triéthylsilane ($Et_3SiH$)**

Le même mode opératoire employé pour la dépolymérisation de la lignine issue du Hêtre Commun, est utilisé pour la dépolymérisation de la lignine issue de l'Eucalyptus. De même, les produits obtenus dans les deux cas sont similaires. Parmi les produits les plus volatils, les produits de formules **IIa, IIb, IIc** et **IId** sont identifiés par RMN et par GC-MS. Le rapport molaire **IIc**/**IIa** est de 76/24 respectivement d'après l'analyse GC-MS.

**EXEMPLE 7 : Dépolymérisation de la lignine issue du Thuya géant (*Thuja plicata*) (extraite par le procédé AVIDEL) en utilisant le triéthylsilane ($Et_3SiH$)**

Le même mode opératoire employé pour la dépolymérisation de la lignine issue de l'Eucalyptus *(Eucalyptus camaldulensis),* est utilisé pour la dépolymérisation de la lignine issue du Thuya géant. De même, les produits obtenus dans les deux cas sont similaires. Parmi les produits les plus volatils, le produit de formules **IIa** et **IIb** ont été identifiés par RMN et par GC-MS comme indiqué dans le Tableau 1.

**EXEMPLE 8 : Dépolymérisation de la lignine issue du mélange de sciure de bois F315 (extraite par le procédé AVIDEL) en utilisant le tétraméthyldisiloxane (TMDS)**

La dépolymérisation de la lignine est effectuée avec la lignine issue du mélange de bois F315 (mélange de sciure de bois commercialisé par la société SPPS extraits d'espèces appartenant à la famille des Pinacées).

Dans le cas où TMDS (tétramethyldisiloxane) est utilisé comme silane, il y a possibilité de formation de gel, ce qui rend la réaction très difficile. Pour ce faire deux solutions peuvent être envisagées : la dilution de la solution 3 à 4 fois en employant le $CH_2Cl_2$ comme solvant ou bien l'utilisation du benzène ou du toluène comme solvant. Toutefois la

réaction sera plus lente dans les deux cas envisagés. Si la réaction se réalise dans $CH_2Cl_2$, la concentration en TMDS est de l'ordre 1-3 $mol.L^{-1}$ (concentration calculée sur la base de la moitié du volume final de solvant introduit). 20 % en masse de $B(C_6F_5)_3$ (masse calculée par rapport à la masse de lignine ajoutée) sont nécessaires pour catalyser la réaction. La masse de lignine ajoutée est entre 10 à 30 % de la masse de silane ajouté. Le temps d'ajout du mélange catalyseur-silane s'étale entre 30 et 45 min. Ensuite, la réaction est laissée sous agitation pendant 24 heures à 25°C.

Après la fin de la réaction, les composés volatils ainsi que le solvant sont évaporés sous vide ($10^{-2}$ mbar). Le mélange issu de la dépolymérisation se dégrade lors de sa purification sur colonne de silice et le produit obtenu est hydrolysé en milieu basique, en utilisant un mélange de THF et de $H_2O$ contenant 10 % en masse de NaOH. Après 16 heures d'agitation à 25°C, les composés volatils ainsi que les solvants sont évaporés, et le produit est purifié sur colonne de silice. L'hydrolyse du mélange conduit à des produits de formule (IV).

**EXEMPLE 9 : Dépolymérisation de la lignine issue du mélange de sciure bois commercial F315 (extraite par le procédé AVIDEL) en utilisant ([(POCOP)Ir(H)(acétone)]$^+$B($C_6F_5$)$_4^-$) et le diéthylsilane ($Et_2SiH_2$)**

La dépolymérisation de la lignine issue du mélange de bois F315 (mélange de sciure de bois commercialisé par la société SPPS extraits d'espèces appartenant à la famille des Pinacées) est réalisée en suivant le protocole opératoire général de dépolymérisation décrit ci-dessus.

Dans le cas où le complexe ([(POCOP)Ir(H)(acétone)]$^+$B($C_6F_5$)$_4^-$) est utilisé pour la dépolymérisation de la lignine, le mode opératoire est similaire à celui où le catalyseur employé est $B(C_6F_5)_3$. $Et_2SiH_2$ (5 $mol.L^{-1}$) est utilisé comme silane dans le chlorobenzène. La masse de la lignine correspond à 30 % de la masse de silane ajouté. La réaction se réalise en présence de 25 % en masse de catalyseur (masse calculée par rapport à la masse de lignine ajoutée). Le temps d'ajout du silane et du catalyseur est de 30 min. Le temps de réaction est de l'ordre de 24 heures. Ensuite, le solvant et les volatiles sont évaporés, et le liquide visqueux obtenu est purifié sur colonne de silice (voir mode opératoire général). L'hydrolyse des produits issus de la colonne est réalisée en agitant les produits pendant 16 heures dans une solution de 2 M de HCl dans le THF. Enfin, les différents polyols correspondants sont obtenus par évaporation du solvant et des volatils sous vide. L'hydrolyse du mélange conduit à des produits de formule (IV).

**Exemple 10 : Dépolymérisation de la lignine issue du mélange de bois F315 (riche en motif G) (extraite par l'éthanol) avec le triéthylsilane ($Et_3SiH$)**

La lignine issue du mélange de bois F315 (mélange de sciure de bois commercialisé par la société SPPS extraits d'espèces appartenant à la famille des Pinacées) a été extraite par l'éthanol et en présence de l'acide chlorhydrique en quantité catalytique, selon la méthode décrite par S. Bauer, H. Sorek, V. D. Mitchell, A. B. Ibáñez, D. E. Wemmer, J. Agric. Food Chem. 2012, 60, pages 8203-8212.

Le même mode opératoire employé pour la dépolymérisation de la lignine issue du Platane est utilisée. Cette méthode conduit à une solubilisation totale de la lignine ainsi que l'obtention d'un mélange de produits.

Parmi les produits les plus volatils, **IIa** et **IIb** sont identifiés par RMN et par GC-MS comme indiqué dans le Tableau 1. L'hydrolyse du mélange conduit à des produits de formule (IV).

**EXEMPLE 11 : Dépolymérisation de la lignine issue du mélange de bois F315 (riche en motif G) (extraite par le méthanol) avec le triéthylsilane ($Et_3SiH$)**

La lignine du mélange de bois F315 (mélange de sciure de bois commercialisé par la société SPPS extraits d'espèces appartenant à la famille des Pinacées) a été extraite par le méthanol, selon la méthode décrite par K. Barta, G. R. Warner, E. S. Beach, P. T. Anastas, Green Chem., 2014, 16, pages 191-196.

Le même mode opératoire employé pour la dépolymérisation de la lignine issue du Platane, est utilisée. Cette méthode conduit à une solubilisation totale de la lignine et sa depolymérisation générant un mélange de produits.

Parmi les produits les plus volatils **IIa** et **IIb** sont identifiés par RMN et par GC-MS comme indiqué dans le Tableau 1. L'hydrolyse du mélange conduit à des produits de formule (IV).

**EXEMPLE 12 : Dépolymérisation de la lignine issue du mélange de bois** F315 **(riche en motif G) (extraite par l'acétone) avec le triéthylsilane ($Et_3SiH$)**

La lignine du mélange de bois F315 (mélange de sciure de bois commercialisé par la société SPPS extraits d'espèces appartenant à la famille des Pinacées) a été extraite par l'acétone et en présence de l'acide chlorhydrique en quantité catalytique, selon la méthode décrite par S. Bauer, H. Sorek, V. D. Mitchell, A. B, Ibáñez, D. E. Wemmer, J. Agric. Food Chem. 2012, 60, pages 8203-8212.

Le même mode opératoire employé pour la dépolymérisation de la lignine issue du Platane, est utilisée. Cette

méthode conduit à une solubilisation totale de la lignine ainsi que l'obtention d'un mélange de produits de formule générale **II**.

Parmi les produits les plus volatils, **IIb** est identifié par RMN et par GC-MS comme indiqué dans le Tableau 1. L'hydrolyse du mélange conduit à des produits de formule (IV).

**EXEMPLE 13 (comparatif) : Dépolymérisation avec le triéthylsilane ($Et_3SiH$) de la lignine commerciale (Aldrich : lignine Kraft) issu de bois tendre puis désulfurée à l'aide de la soude**

Le même mode opératoire employé pour la dépolymérisation de la lignine issue de l'Eucalyptus (*Eucalyptus camaldulensis),* est utilisé pour la dépolymérisation de la lignine issue du procédé Kraft et ayant un taux de soufre de 3,76 % en masse par rapport à la masse totale de la lignine. Aucune solubilisation ou dépolymérisation n'a été observée dans le cas de cette lignine. Dans le cas où ce même échantillon de lignine est retraité par le procédé AVIDEL, le taux de soufre atteint 3% en masse par rapport à la masse totale de la lignine, mais la dépolymérisation ne se réalise toujours pas. Ceci implique que la présence de soufre dans le milieu réactionnel joue un rôle crucial dans la désactivation de la réaction.

Les composés de formules (IV) obtenus après l'hydrolyse des composés silylés issus de la dépolymérisation de la lignine sont de formule (IV)

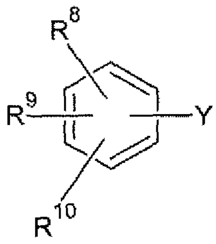

(IV)

dans laquelle

- $R^8$, $R^9$, $R^{10}$, représentent, indépendamment l'un de l'autre, un atome d'hydrogène, un groupe hydroxyle ;
- Y représente un groupe alkyle, un groupe alcényle, un groupe alcynyle, un groupe carbonyle $-CR^4=O$ avec $R^4$ représentant un atome d'hydrogène, un groupe alkyle, un groupe hydroxyle, un groupe alkoxy,

lesdits groupes alkyle, alcényle et alcynyle étant éventuellement substitués.

Le Tableau 1 récapitule les résultats de dépolymérisation des lignines des exemples indiqués ci-dessus. Dans le Tableau 1 :

% de lignine extraite signifie le pourcentage massique de lignine extrait par rapport à la masse de bois initialement employé ;

% massique signifie le pourcentage massique de l'espèce par rapport à la masse de lignine initiale introduite évalué par un étalonnage externe de la GC-MS par les mêmes molécules analysées.

Les conditions opératoires appliquées pour obtenir les résultats du Tableau 1 sont les suivantes : Lignine, $Et_3SiH$ (275 à 320 % masse/masse lignine), $B(C_6F_5)_3$ (15 à 25 % masse/masse lignine), $CH_2Cl_2$ (995 % masse/masse lignine), 25°C, 16 heures.

| Source de lignine | Méthode d'extraction de la lignine utilisée | % massique de lignine extraite | % massique IIa | % massique IIc | % massique IIb | % massique IId |
|---|---|---|---|---|---|---|
| F315 (bois mou) | reflux de méthanol | 2 | 15 | - | 1 | - |
| Pin parasol (bois mou) | AVIDEL | 8 | 16 | - | - | - |

(suite)

| Source de lignine | Méthode d'extraction de la lignine utilisée | % massique de lignine extraite | % massique IIa | % massique IIc | % massique IIb | % massique IId |
|---|---|---|---|---|---|---|
| Thuya géant (bois dur) | AVIDEL | 7 | 8 | - | 2 | |
| Epicéa commun (bois mou) | AVIDEL | 6 | 16 | - | 18 | - |
| F315 (bois - mou) | reflux d'éthanol | 3 | 13 | - | 15 | - |
| F315 (bois mou) | reflux d'acétone | 2 | - | - | 4 | - |
| Hêtre commun (bois dur) | AVIDEL | 14 | 13 | 22 | - | - |
| Peuplier d'Italie (bois dur) | AVIDEL | 17 | 19 | 21 | - | - |
| Bouleau pendant (bois dur) | AVIDEL | 13 | 10 | 26 | - | - |
| Chêne vert - (bois dur) | AVIDEL | 12 | 6 | 37 | - | 13 |
| Palmier-dattier (bois dur) | AVIDEL | 10 | 3 | 6 | 10 | 79 |
| Eucalyptus (bois dur) | AVIDEL | 9 | 8 | 30 | 17 | 35 |
| Prune verte (bois dur) | AVIDEL | 18 | 20 | - | 3 | 26 |
| Platane (bois dur) | AVIDEL | 10 | - | 15,6 | 9 | 65 |
| Cèdre du Liban (bois mou) | AVIDEL | 6 | 14 | - | 3 | - |
| Sapin Nordmann (bois mou) | AVIDEL | 20 | 2 | - | - | - |
| Ebène du Gabon (bois dur) | AVIDEL | 7 | - | - | 6 | - |

**Protocole expérimental d'hydrolyse de composés aromatiques silylés issus de la dépolymérisation réductrice de la lignine**

A une solution de **IIa** (380,7 mg; 1,0 mmol, 1 équivalent) dans 4 mL de THF, $n\text{Bu}_4\text{NF}.3\text{H}_2\text{O}$ (315,5 mg, 2,1 mmol, 2,1 équiv) a été ajouté lentement (environ 5 min) sous argon. La solution a été agitée pendant 1 h à 20 °C. Ensuite, les volatils ont été évaporés sous vide et 4 mL de dichlorométhane ont été ajoutés. Enfin, le composé **IIa** a été purifié

sur colonne de silice en utilisant un gradient d'élution allant de 50% dichlorométhane à 50% acétate d'éthyle. L'évaporation des solvants conduit à l'obtention de 4-propylbenzene-1,2-diol (141,5 mg; 0,9 mmol; 84%) sous forme d'une huile incolore.

Le Tableau 2 récapitule les résultats de de l'hydrolyse des molécules aromatiques silylées **IIa-IIf** issues de la dépolymérisation réductrice de la lignine des lignines des exemples indiqués ci-dessus.

Tableau 2 :

| Molécule aromatique silylée | Quantité de TBAF (équiv.) | Apparence | Rendement isolé (%) |
|---|---|---|---|
| **IIa** | 2,1 | Huile incolore | 84 |
| **IIb** | 3,1 | Huile incolore | 86 |
| **IIc** | 3,1 | Poudre blanche ou cristaux incolores | 94 |
| **IId** | 4,1 | Gomme blanche | 82 |
| **IIe** | 1,1 | Huile incolore | 77 |
| **IIf** | 2,1 | Poudre blanche | 92 |

Après l'hydrolyse, toutes les liaisons O-Si se transforment en O-H.

## Revendications

1. Procédé de dépolymérisation de la lignine en molécules contenant de 1 à 2 cycles aromatiques, par clivage sélectif de la liaison carbone $sp^3$-oxygène des alkylaryle éthers du type β-O-4, α-O-4, β-5, β-1, β-β présents dans la lignine, **caractérisé en ce que** l'on fait réagir, en présence d'un catalyseur,

   - une lignine avec un taux de soufre comme défini ci-dessous :
   0 ≤ taux de soufre de la lignine < 1,5 % en masse, par rapport à la masse totale de la lignine, avec
   - un composé silane de formule (I)

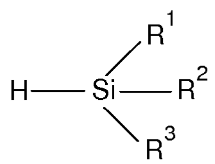

(I)

   dans laquelle

   ▪ $R^1$, $R^2$ et $R^3$ représentent, indépendamment l'un de l'autre, un atome d'hydrogène, un atome d'halogène, un groupe hydroxyle, un groupe alkyle, un groupe alcényle, un groupe alcynyle, un groupe alkoxy, un groupe aryloxy, un groupe silylé, un groupe siloxy, un groupe aryle, un groupe amino, lesdits groupes alkyle, alcényle, alcynyle, alkoxy, silylé, siloxy, aryle et amino étant éventuellement substitués, ou
   ▪ $R^3$ est tel que défini ci-dessus et $R_1$ et $R_2$, pris ensemble avec l'atome de silicium auquel ils sont liés forment un hétérocycle silylé éventuellement substitué ; et

   **en ce que** les molécules aromatiques contenant 1 à 2 cycles aromatiques présentent une masse molaire moyenne en poids inférieure à 1500 g/mol pour les molécules sous forme silylée ou de masse molaire moyenne en poids égale ou inférieure à 400g/mol sous forme non silylée, soit un degré de polymérisation entre 1 et 2 unités monomères ; et
   **en ce que** la lignine est extraite par les procédés organosolv.

2. Procédé selon l'une des revendications 1, **caractérisé en ce que** la lignine est extraite d'une espèce végétale sélectionnée de façon à avoir :

- au moins 10 % en masse de la lignine par rapport à la masse totale de l'échantillon de l'espèce végétale sélectionnée ;
- au moins 30 % de liaisons clivables par rapport au nombre total de liaisons présentes entre les motifs monomères dans la lignine ; et
- au moins 50 % de résidu p-hydroxyphényle (H), guaiacyle (G), et syringyl (S) du nombre total de résidus présents dans la lignine utilisée.

**3.** Procédé selon l'une quelconque des revendications 1 ou 2, **caractérisé en ce que** dans le composé silane de formule (I), $R^1$, $R^2$ et $R^3$ représentent indépendamment l'un de l'autre, un atome d'hydrogène ; un groupe alkyle choisi parmi les groupes méthyle, éthyle, propyle, butyle, pentyle, hexyle, heptyle et leurs isomères ramifiés ; un groupe alkoxy dont le groupe alkyle est choisi parmi les groupes méthyle, éthyle, propyle, butyle, pentyle, hexyle, heptyle et leurs isomères ramifiés ; un groupe aryle choisi parmi les groupes benzyle et phényle ; un groupe silylé choisi parmi le polydiméthylsiloxane (PDMS) et le polyméthylhydroxysiloxane (PMHS) et le tétraméthyldisiloxane (TMDS).

**4.** Procédé selon l'une quelconque des revendications 1 à 3, **caractérisé en ce que** le catalyseur est un catalyseur organique choisi parmi :

- les carbocations choisis parmi le cation trityle ($(C_6H_5)_3C^+$), le tropilium $(C_7H_7)^+$, le cation benzylique ($C_6H_5CH_2^+$), le cation allylique ($CH_3$-$CH^+$-$CH$=$CH_2$), le méthylium ($CH_3^+$), le cyclopropylium ($C_3H_5^+$), le carbocation cyclopropylique choisi parmi le carbocation diméthyle cyclopropylique et le carbocation dicyclopropylique, 1' acylium $(R^1\text{-}C{=}O)^+$ avec $R^1$ choisi parmi le méthyle, le propyle et le benzyle, le cation benzénium $(C_6H_5)^+$, le cation norbornyle $(C_7H_{11})^+$ ;
- les oxoniums choisis parmi $(CH_3)_3O^+BF_4^-$ et $(CH_3CH_2)_3O^+BF_4^-$ ;
- un ion silylium $(R^1)_3Si^+$ avec $R^1$ tel que défini à la revendication 1, choisi parmi $Et_3Si^+$ et $Me_3Si^+$ ;
- les cations disilyles ayant un hydrure pontant choisis parmi les formules indiquées ci-dessous

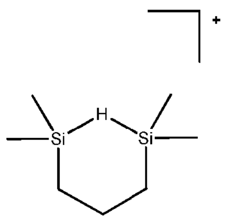
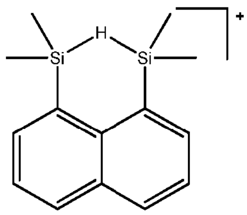
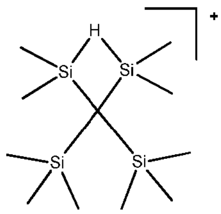

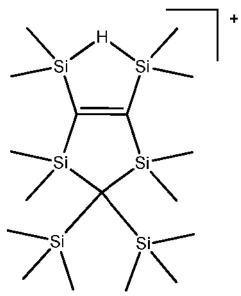

avec le contre ion dudit ion silylium, desdits carbocations et desdits cations disilyles étant

- un halogénure choisi parmi $F^-$, $Cl^-$, $Br^-$ et $I^-$ ; ou
- un anion choisi parmi $BF_4^-$, $SbF_6^-$, $B(C_6F_5)_4^-$, $B(C_6H_5)_4^-$, $CF_3SO_3^-$, $PF_6^-$.

**5.** Procédé selon l'une quelconque des revendications 1 à 3, **caractérisé en ce que** le catalyseur est un catalyseur organométallique choisi parmi :

- les complexes d'iridium de formule (III)

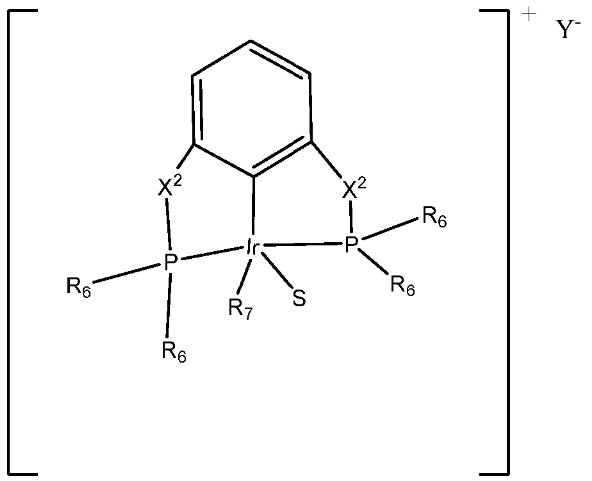

Formule (III)

dans laquelle

- $R^6$ représente un groupe alkyle ou aryle ;
- $R^7$ représente un atome d'hydrogène ou un groupe alkyle ; et
- $X^2$ représente un groupe $-CH_2-$ ou un atome d'oxygène ;
- Y représente un contre ion choisi parmi $B(C_6F_5)_4$ et $B(C_6H_5)_4$ ;
- S représente une molécule de solvant, coordonnée au complexe, choisi parmi le diméthylesulfoxyde (DMSO), l'acétonitrile ($CH_3CN$) et l'acétone ($CH_3COCH_3$) ; et

▪ les complexes de ruthénium de formule (V)

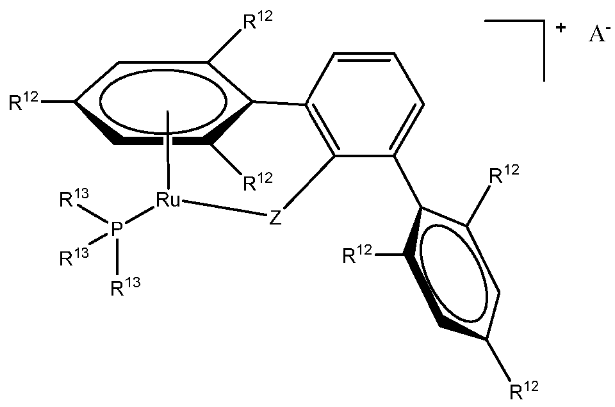

Formule (V)

dans laquelle

- $R^{12}$ représente un atome d'hydrogène ou un groupe alkyle ;
- $R^{13}$ représente un aryle ou un groupe alkyle, lesdits groupes aryle et alkyle étant éventuellement substitués ;
- Z représente un groupe $-CH_2-$, un atome d'oxygène ou un atome de soufre ;
- $A^-$ représente un contre ion choisi parmi $B(C_6F_5)_4^-$ et $[CHB_{11}H_5Cl_6]^-$.

**6.** Procédé selon l'une quelconque des revendications 1 à 3 et 5, **caractérisé en ce que** le catalyseur organométallique est choisi parmi

▪ le complexe d'iridium $[(POCOP)Ir(H)(acétone)]^+B(C_6F_5)_4^-$ avec (POCOP) représentant le 2,6-bis(di-tert-butylphosphinito)phényle ; et

- le complexe de ruthénium de formule (V) dans laquelle

  - $R^{12}$ représente un groupe méthyle ;
  - $R^{13}$ représente *p*-$FC_6H_4$ ;
  - Z représente un atome de soufre ;

$A^-$ représente $B(C_6F_5)_4^-$.

**7.** Procédé selon l'une quelconque des revendications 1 à 3, **caractérisé en ce que** le catalyseur est de type acide de Lewis choisi parmi :

- les composés de bore choisis parmi $BF_3$, $BF_3(Et_2O)$, $BCl_3$, $BBr_3$, le triphényle hydroborane, le tricyclohexyle hydroborane, $B(C_6F_5)_3$, le B-méthoxy-9-borabicyclo[3.3.1]nonane (B-méthoxy-9-BBN), le B-benzyl-9-borabicyclo[3.3.1]nonane (B-benzyl-9-BBN) ;
- le composés borénium Me-TBD-BBN$^+$, les dérivés borenium ferrocène répondant à la formule

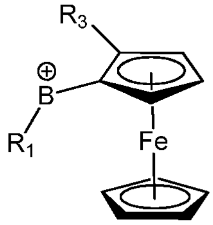

borenium ferrocène

dans laquelle $R^1$, est un groupe phényle et $R^3$ est le 3,5-diméthylpyridyle ;
- les composés de l'aluminium choisis parmi $AlCl_3$, $AlBr_3$, l'isopropoxyde d'aluminium $Al(O\text{-}i\text{-}Pr)_3$, l'éthanoate d'aluminium ($Al(C_2H_3O_2)$), le sel de Krossing $[Ag(CH_2Cl_2)]\{Al[OC(CF_3)_3]_4\}$, le $Li\{Al[OC(CF_3)_3]_4\}$, $Et_2Al^+$ ;
- les composés d'indium choisis parmi $InCl_3$, $In(OTf)_3$ ;
- les composés de fer choisis parmi $FeCl_3$, $Fe(OTf)_3$ ;
- les composés de l'étain choisis parmi $SnCl_4$, $Sn(OTf)_2$ ;
- les composés du phosphore tels que $PCl_3$, $PCl_5$, $POCl_3$ ;
- les composés trifluorométhanesulfonates ou triflates ($CF_3SO_3^-$) de métaux de transitions et des lanthanides choisis parmi le triflate de scandium, le triflate de ytterbium, le triflate d'yttrium, le triflate de cérium, le triflate de samarium, le triflate de niodinium.

**8.** Procédé selon l'une quelconque des revendications 1 à 3 et 7, **caractérisé en ce que** le catalyseur est choisi parmi $BF_3$ ; $InCl_3$ ; le triphénylcarbénium tetrakis(perfluorophenyl)borate $[(Ph)_3C^+B(C_6F_5)_4^-, B(C_6F_5)_3]$.

**9.** Procédé selon l'une quelconque des revendications 1 à 8, **caractérisé en ce que** la réaction est effectuée sous une pression d'un ou d'un mélange de gaz inerte(s) choisi(s) parmi l'azote et l'argon, ou des gaz générés par le procédé notamment du méthane et d'hydrogène, ladite pression étant comprise entre 0,2 et 50 bars, bornes incluses.

**10.** Procédé selon l'une quelconque des revendications 1 à 9, **caractérisé en ce que** la réaction est effectuée dans un ou un mélange d'au moins deux solvant(s) choisi(s) parmi :

- les éthers silylés choisis parmi le 1,1,1,3,3,3-hexaméthyldisiloxane ($(Me_3Si)_2O$), le 1,1,1,3,3,3-hexaéthyldisiloxane ($(Et_3Si)_2O$) ;
- les hydrocarbures choisis parmi le benzène, le toluène, le pentane et l'hexane ;
- les sulfoxydes choisis parmi le diméthylesulfoxyde (DMSO) ;
- les halogénures d'alkyle choisis parmi le chloroforme, le chlorure de méthylène, le chlorobenzène, le dichlorobenzène.

**11.** Procédé selon l'une quelconque des revendications 1 à 10, **caractérisé en ce que** le rapport massique entre le composé silane de formule (I) et la lignine est compris entre 0,5 et 6, bornes incluses.

12. Procédé selon l'une quelconque des revendications 1 à 11, **caractérisé en ce que** la quantité de catalyseur est de 0,001 à 1 équivalent en masse, bornes incluses, par rapport à la masse initiale de lignine.

13. Utilisation d'un procédé de dépolymérisation de la lignine selon l'une quelconque des revendications 1 à 12, dans la fabrication de combustibles, de composants électroniques, de polymères plastiques, de caoutchouc, de médicaments, de vitamines, de produits cosmétiques, de parfums, de produits alimentaires, de fils et fibres synthétiques, de cuirs synthétiques, de colles, de pesticides, d'engrais.

## Patentansprüche

1. Verfahren zur Depolymerisation von Lignin zu Molekülen, die 1 bis 2 aromatische Zyklen enthalten, durch selektive Spaltung der $sp^3$-Sauerstoff-Kohlenstoffbindung von Alkylarylethern vom Typ β-O-4, α-O-4, β-5, β-1, β-β, die in Lignin vorhanden sind, **dadurch gekennzeichnet, dass** in Gegenwart eines Katalysators reagieren gelassen werden

   - ein Lignin mit einem Schwefelgehalt wie nachstehend definiert:
   0 <Schwefelgehalt von Lignin < 1,5 Massen-% bezogen auf die Gesamtmasse von Lignin mit
   - einer Silanverbindung der Formel (I)

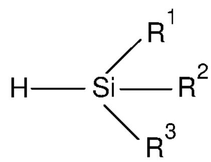

(I),

   in welcher

   - $R^1$, $R^2$ und $R^3$ unabhängig voneinander ein Wasserstoffatom, ein Halogenatom, eine Hydroxylgruppe, eine Alkylgruppe, eine Alkenylgruppe, eine Alkinylgruppe, eine Alkoxygruppe, eine Aryloxygruppe, eine Silylgruppe, eine Siloxygruppe, eine Arylgruppe, eine Aminogruppe darstellen, wobei die Alkyl-, Alkenyl-, Alkinyl-, Alkoxy-, Silyl-, Siloxy-, Aryl- und Aminogruppen gegebenenfalls substituiert sind, oder
   - $R^3$ wie oben definiert ist, und $R_1$ und $R_2$ zusammen mit dem Siliziumatom, an das sie gebunden sind, einen gegebenenfalls substituierten silylierten Heterozyklus bilden, und

   dass die aromatischen Moleküle, die 1 bis 2 aromatische Zyklen enthalten, eine gewichtsmittlere Molmasse von weniger als 1500 g/mol für die Moleküle in silylierter Form oder eine gewichtsmittlere Molmasse gleich oder weniger als 400 g/mol in nicht silylierter Form, also einen Polymerisationsgrad zwischen 1 und 2 Monomereinheiten, aufweisen; und dass das Lignin durch Organosolv-Verfahren extrahiert wird.

2. Verfahren nach einem der Ansprüche 1, **dadurch gekennzeichnet, dass** das Lignin aus einer Pflanzenart extrahiert wird, die so ausgewählt ist, dass es hat:

   - mindestens 10 Massen-% des Lignins bezogen auf die Gesamtmasse der Probe der ausgewählten Pflanzenart;
   - mindestens 30 % an spaltbaren Bindungen bezogen auf die Gesamtzahl der zwischen den Monomermustern im Lignin vorhandenen Bindungen; und
   - mindestens 50 % p-Hydroxyphenyl- (H), Guaiacyl- (G) und Syringyl- (S) Rest der Gesamtzahl der im verwendeten Lignin vorhandenen Reste.

3. Verfahren nach einem der Ansprüche 1 oder 2, **dadurch gekennzeichnet, dass** in der Silanverbindung der Formel (I) $R^1$, $R^2$ und $R^3$ unabhängig voneinander ein Wasserstoffatom; eine Alkylgruppe, die ausgewählt ist aus Methyl, Ethyl, Propyl, Butyl, Pentyl, Hexyl, Heptyl und deren verzweigten Isomeren; eine Alkoxygruppe, deren Alkylgruppe ausgewählt ist aus Methyl-, Ethyl-, Propyl-, Butyl-, Pentyl-, Hexyl-, Heptylgruppen und deren verzweigten Isomeren; eine Arylgruppe, die ausgewählt ist aus Benzyl- und Phenylgruppen; eine Silylgruppe, die ausgewählt ist aus Polydimethylsiloxan (PDMS) und Polymethylhydroxysiloxan (PMHS) und Tetramethyldisiloxan (TMDS), darstellen.

4. Verfahren nach einem der Ansprüche 1 bis 3, **dadurch gekennzeichnet, dass** der Katalysator ein organischer

Katalysator ist, der ausgewählt ist aus:

- Carbokationen, die ausgewählt sind aus dem Tritylkation ($(C_6H_5)_3C^+$), Tropilium $(C_7H_7)^+$, dem Benzylkation ($C_6H_5CH_2^+$), dem Allylkation ($CH_3$-$CH^+$-$CH$=$CH_2$), Methylium ($CH_3^+$), Cyclopropylium ($C_3H_5^+$), dem Cyclopropylcarbokation, das ausgewählt ist aus dem Dimethylcyclopropylcarbokation und dem Dicyclopropylcarbokation, Acylium $(R^1\text{-}C{=}O)^+$, wobei $R^1$ ausgewählt ist aus Methyl, Propyl und Benzyl, dem Benzoliumkation $(C_6H_5)^+$, dem Norbornylkation $(C_7H_{11})^+$;
- Oxoniums, die ausgewählt sind aus $(CH3)_3O^+BF_4^-$ und $(CH_3CH_2)_3O^+BF_4^-$;
- ein Silyliumion $(R^1)_3Si^+$, wobei $R^1$ wie im Anspruch 1 definiert ist, wobei es ausgewählt ist aus $Et_3Si^+$ und $Me_3Si^+$;
- Disilylkationen mit einem Brückenhydrid, die ausgewählt sind aus den unten angegebenen Formeln

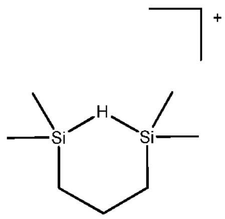

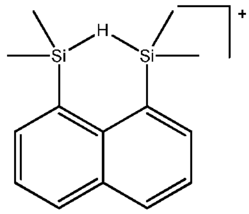

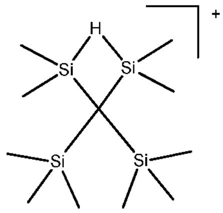

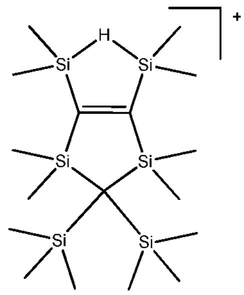

wobei das Gegenion des Silyliumions, der Carbokationen und der Disilylkationen ist

- ein Halogenid, das ausgewählt ist aus $F^-$, $Cl^-$, $Br^-$ und $I^-$; oder
- ein Anion, das ausgewählt ist aus $BF_4^-$, $SbF_6^-$, $B(C_6F_5)_4^-$, $B(C_6H_5)_4^-$, $CF_3SO_3^-$, $PF_6^-$.

**5.** Verfahren nach einem der Ansprüche 1 bis 3, **dadurch gekennzeichnet, dass** der Katalysator ein metallorganischer Katalysator ist, der ausgewählt ist aus:

- Iridiumkomplexen der Formel (III)

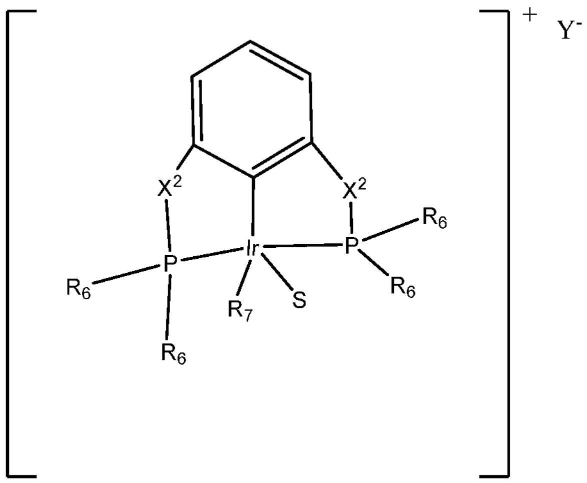

Formel (III),

in der

- $R^6$ eine Alkyl- oder Arylgruppe darstellt;
- $R^7$ ein Wasserstoffatom oder eine Alkylgruppe darstellt; und
- $X^2$ eine $-CH_2-$ Gruppe oder ein Sauerstoffatom darstellt;
- Y ein Gegenion darstellt, das ausgewählt ist aus $B(C_6F_5)_4$ und $B(C_6H_5)_4$;
- S ein an den Komplex koordiniertes Lösungsmittelmolekül darstellt, das ausgewählt ist aus Dimethylsulfoxid (DMSO), Acetonitril ($CH_3CN$) und Aceton ($CH_3COCH_3$); und

▪ Rutheniumkomplexen der Formel (V)

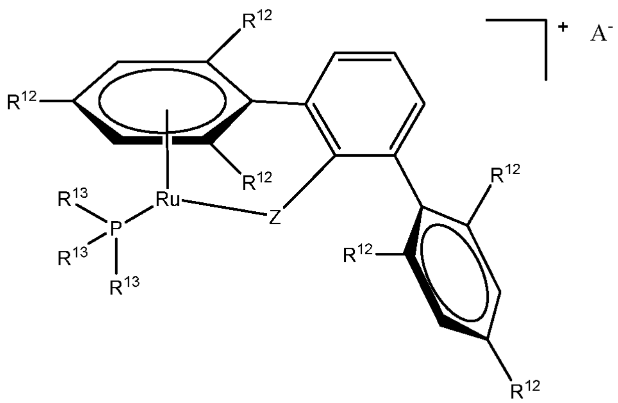

Formel (V)

in der

- $R^{12}$ ein Wasserstoffatom oder eine Alkylgruppe darstellt;
- $R^{13}$ eine Aryl- oder Alkylgruppe darstellt, wobei die Aryl- und Alkylgruppen gegebenenfalls substituiert sind;
- Z eine $-CH_2^-$ Gruppe, ein Sauerstoffatom oder ein Schwefelatom darstellt;
- $A^-$ ein Gegenion darstellt, das ausgewählt ist aus $B(C_6F_5)_4^-$ und $[CHB_{11}H_5Cl_6]^-$.

**6.** Verfahren nach einem der Ansprüche 1 bis 3 und 5, **dadurch gekennzeichnet, dass** der metallorganische Katalysator ausgewählt ist aus

▪ Iridiumkomplex $[(POCOP)Ir(H)(Aceton)]^+B(C_6F_5)_4^-$, wobei (POCOP) 2,6-Bis(di-tert-butylphosphinito)phenyl darstellt; und

- Rutheniumkomplex der Formel (V), in dem

  - $R^{12}$ eine Methylgruppe darstellt;
  - $R^{13}$ *p*-$FC_6H_4$ darstellt;
  - Z ein Schwefelatom darstellt;
  - $A^-$ $B(C_6F_5)_4^-$ darstellt.

**7.** Verfahren nach einem der Ansprüche 1 bis 3, **dadurch gekennzeichnet, dass** der Katalysator vom Lewis-Säure-Typ ist, der ausgewählt ist aus:

- Borverbindungen, die ausgewählt sind aus $BF_3$, $BF_3(Et_2O)$, $BCl_3$, $BBr_3$, Triphenylhydroboran, Tricyclohexylhydroboran, $B(C_6F_5)_3$, B-Methoxy-9-borabicyclo[3.3.1]nonan (B-methoxy-9-BBN), B-Benzyl-9-borabicyclo[3.3.1]nonan (B-Benzyl-9-BBN);
- Boreniumverbindungen Me-TBD-$BBN^+$, Boreniumferrocenderivaten, die sind gemäß der Formel:

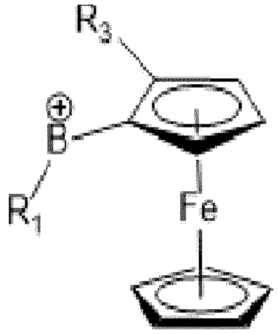

Boreniumferrocen

wobei $R^1$ eine Phenylgruppe ist, und $R^3$ 3,5-Dimethylpyridyl ist;

  - Aluminiumverbindungen, die ausgewählt sind aus $AlCl_3$, $AlBr_3$, Aluminiumisopropoxid $Al(O\text{-}i\text{-}Pr)_3$, Aluminiumethanoat ($Al(C_2H_3O_2)$), Krossing-Salz $[Ag(CH_2Cl_2)]\{Al[OC(CF_3)_3]_4\}$, $Li\{Al[OC(CF_3)_3]_4\}$, $Et_2Al^+$;
  - Indiumverbindungen, die ausgewählt sind aus $InCl_3$, $In(OTf)_3$;
  - Eisenverbindungen, die ausgewählt sind aus $FeCl_3$, $Fe(OTf)_3$;
  - Zinnverbindungen, die ausgewählt sind aus $SnCl_4$, $Sn(OTf)_2$;
  - Phosphorverbindungen wie $PCl_3$, $PCl_5$, $POCl_3$;
  - Trifluormethansulfonat- oder Triflat- ($CF_3SO_3^-$) Verbindungen von Übergangsmetallen und Lanthaniden, die ausgewählt sind aus Scandiumtriflat, Ytterbiumtriflat, Yttriumtriflat, Certriflat, Samariumtriflat, Niodiniumtriflat.

**8.** Verfahren nach einem der Ansprüche 1 bis 3 und 7, **dadurch gekennzeichnet, dass** der Katalysator ausgewählt ist aus $BF_3$; $InCl_3$; Triphenylcarbeniumtetrakis(perfluorphenyl)borat $[(Ph)_3C^+B(C_6F_5)_4^-$, $B(C_6F_5)_3]$.

**9.** Verfahren nach einem der Ansprüche 1 bis 8, **dadurch gekennzeichnet, dass** die Reaktion unter einem Druck von einem oder einem Gemisch von Inertgasen durchgeführt wird, das (die) ausgewählt ist (sind) aus Stickstoff und Argon oder durch das Verfahren erzeugte Gase, insbesondere Methan und Wasserstoff, wobei der Druck zwischen 0,2 und 50 bar einschließlich der Grenzwerte liegt.

**10.** Verfahren nach einem der Ansprüche 1 bis 9, **dadurch gekennzeichnet, dass** die Reaktion in einem oder einem Gemisch von mindestens zwei Lösungsmitteln durchgeführt wird, das (die) ausgewählt ist (sind) aus:

- Silylethern, die ausgewählt sind aus 1,1,1,3,3,3-Hexamethyldisiloxan ($(Me_3Si)_2O$), 1,1,1,3,3,3-Hexaethyldisiloxan ($(Et_3Si)_2O$);
- Kohlenwasserstoffen, die ausgewählt sind aus Benzol, Toluol, Pentan und Hexan;
- Sulfoxiden, die ausgewählt sind aus Dimethylsulfoxid (DMSO);
- Alkylhalogeniden, die ausgewählt sind aus Chloroform, Methylenchlorid, Chlorbenzol, Dichlorbenzol.

**11.** Verfahren nach einem der Ansprüche 1 bis 10, **dadurch gekennzeichnet, dass** das Massenverhältnis zwischen der Silanverbindung der Formel (I) und dem Lignin zwischen 0,5 und 6 einschließlich der Grenzwerte liegt.

**12.** Verfahren nach einem der Ansprüche 1 bis 11, **dadurch gekennzeichnet, dass** die Katalysatormenge 0,001 bis 1 Massenäquivalent einschließlich der Grenzwerte bezogen auf die Ausgangsmasse von Lignin beträgt.

**13.** Verwendung eines Verfahrens zur Depolymerisation von Lignin nach einem der Ansprüche 1 bis 12 bei der Herstellung von Kraftstoffen, elektronischen Bauteilen, Kunststoffpolymeren, Gummi, Arzneimitteln, Vitaminen, Kosmetika, Parfums, Lebensmitteln, synthetischen Fäden und Fasern, synthetischen Ledern, Klebstoffen, Pestiziden, Düngemitteln.

## Claims

**1.** Method of depolymerising lignin into molecules containing 1 to 2 aromatic cycles, by selective cleavage of the $sp^3$ carbon-oxygen bond of alkylaryl ethers of the β-O-4, α-O-4, β-5, β-1, β-β type present in lignin, **characterised in that** it is made to react, in the presence of a catalyst,

- a lignin with a sulphur ratio as defined below:
$0 \leq$ sulphur ratio of the lignin < 1.5% by mass, with respect to the total mass of the lignin, with
- a silane compound of formula (I)

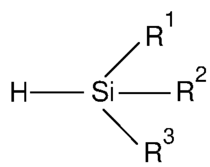

(I)

wherein

- $R^1$, $R^2$ and $R^3$ represent, independently from one another, a hydrogen atom, a halogen atom, a hydroxyl group, an alkyl group, an alkenyl group, an alkynyl group, an alkoxy group, an aryloxy group, a silyl group, a siloxy group, an aryl group, an amino group, said alkyl, alkenyl, alkynyl, alkoxy, silyl, siloxy, aryl and amino groups being optionally substituted, or
- $R^3$ is such as defined above and $R_1$ and $R_2$, taken together with the silicon atom to which they are bonded form an optionally substituted silyl heterocycle; and

**in that** the aromatic molecules containing 1 to 2 aromatic cycles have an average molar mass by weight less than 1500g/mol for molecules in silyl form or average molar mass by weight equal to or less than 400g/mol in non-silyl form, that is a polymerisation degree of between 1 and 2 monomer units; and
**in that** lignin is extracted by organosolv methods.

**2.** Method according to claim 1, **characterised in that** lignin is extracted from a plant species selected so as to have:

- at least 10% by mass of lignin with respect to the total mass of the sample of the plant species selected;
- at least 30% of cleavable bonds with respect to the total number of bonds present between the monomer units in lignin; and
- at least 50% of p-hydroxyphenyl (H), guaiacyl (G), and syringyl (S) residue of the total number of residues present in the lignin used.

**3.** Method according to any one of claims 1 or 2, **characterised in that** in the silane compound of formula (I), $R^1$, $R^2$ and $R^3$ represent independently from one another, a hydrogen atom; an alkyl group chosen from among methyl, ethyl, propyl, butyl, pentyl, hexyl, heptyl groups and their branched isomers; an alkoxyl group of which the alkyl group is chosen from among methyl, ethyl, propyl, butyl, pentyl, hexyl, heptyl groups and their branched isomers; an aryl group chosen from among benzyl and phenyl groups; a silyl group chosen from among polydimethylsiloxane (PDMS) and polymethylhydroxysiloxane (PMHS) and tetramethyldisiloxane (TMDS).

4. Method according to any one of claims 1 to 3, **characterised in that** the catalyst is an organic catalyst chosen from among:

- the carbocations chosen from among trityl cation ($(C_6H_5)_3C^+$), tropilium $(C_7H_7)^+$, benzylic cation ($C_6H_5CH_2^+$), allylic cation ($CH_3$-$CH^+$-$CH=CH_2$), methylium ($CH_3^+$), cyclopropylium ($C_3H_5^+$), cyclopropylic carbocation chosen from among cyclopropylic dimethyl carbocation and dicyclopropylic carbocation, acylium $(R^1$-$C=O)^+$ with $R^1$ chosen from among methyl, propyl and benzyl, benzenium cation $(C_6H_5)^+$, norbornyl cation $(C_7H_{11})^+$;
- oxoniums chosen from among $(CH_3)_3O^+BF_4^-$ and $(CH_3CH_2)_3O^+BF_4^-$;
- a silylium ion $(R^1)_3Si^+$ with $R^1$ such as defined in claim 1, chosen from among $Et_3Si^+$ and $Me_3Si^+$;
- disilyl cations having a bridging hydride chosen from among the formulas indicated below

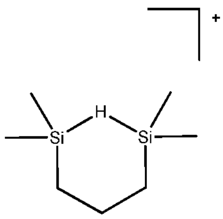
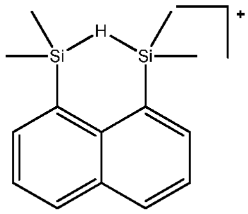
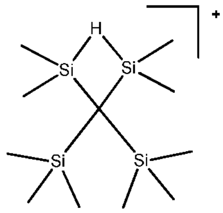

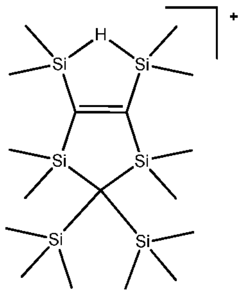

with the counterion of said silylium ion, said carbocations and said disilyl cations being

• a halide chosen from among $F^-$, $Cl^-$, $Br^-$ and $I^-$; or
• an anion chosen from among $BF_4^-$, $SbF_6^-$, $B(C_6F_5)_4^-$, $B(C_6H_5)_4^-$, $CF_3SO_3^-$, $PF_6^-$.

5. Method according to any one of claims 1 to 3, **characterised in that** the catalyst is an organometallic catalyst chosen from among:

- the iridium complexes of formula (III)

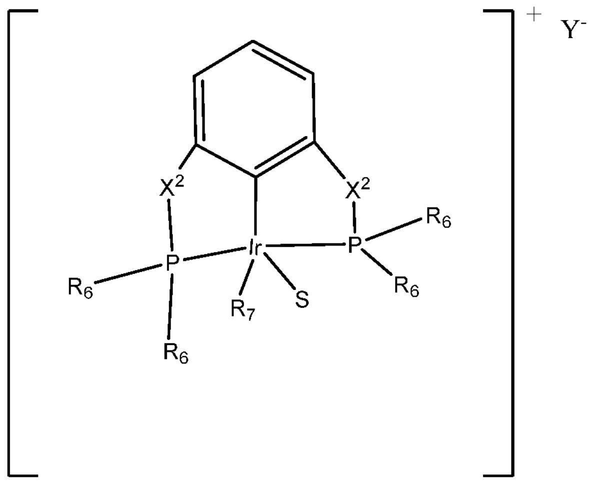

Formula (III)

wherein

- $R^6$ represents an alkyl or aryl group;
- $R^7$ represents a hydrogen atom or an alkyl group; and
- $X^2$ represents a $-CH_2-$ group or an oxygen atom;
- Y represents a counterion chosen from among $B(C_6F_5)_4$ and $B(C_6H_5)_4$;
- S represents a solvent molecule, coordinated with the complex, chosen from among dimethylesulfoxide (DMSO), acetonitrile ($CH_3CN$) and acetone (CH3COCH3); and

- the ruthenium complexes of formula (V)

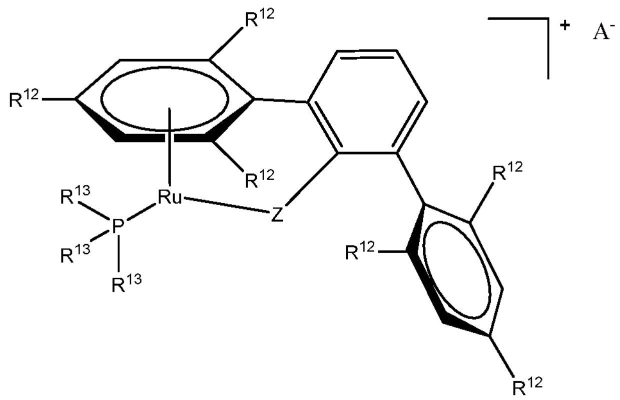

Formula (V)

wherein

- $R^{12}$ represents a hydrogen atom or an alkyl group;
- $R^{13}$ represents an aryl or an alkyl group, said aryl and alkyl groups being possibly substituted;
- Z represents a $-CH_2-$ group, an oxygen atom or a sulphur atom;
- $A^-$ represents a counterion chosen from among $B(C_6F_5)_4^-$ and $[CHB_{11}H_5Cl_6]^-$.

6. Method according to any one of claims 1 to 3 and 5, **characterised in that** the organometallic catalyst is chosen from among

- the iridium complex $[(POCOP)Ir(H)(acetone)]^+B(C_6F_5)_4^-$ with (POCOP) representing 2,6-bis(di-tert-butylphosphinito)phenyl; and

- the ruthenium complex of formula (V) wherein

    - $R^{12}$ represents a methyl group;
    - $R^{13}$ represents *p*-$FC_6H_4$;
    - Z represents a sulphur atom;
    - $A^-$ represents $B(C_6F_5)_4^-$.

7. Method according to any one of claims 1 to 3, **characterised in that** the catalyst is of Lewis acid type chosen from among:

    - boron compounds chosen from among $BF_3$, $BF_3(Et_2O)$, $BCl_3$, $BBr_3$, hydroborane triphenyl, hydroborane tricyclohexyl, $B(C_6F_5)_3$, B-methoxy-9-borabicyclo[3.3.1]nonane (B-methoxy-9-BBN), B-benzyl-9-borabicyclo[3.3.1]nonane (B-benzyl-9-BBN);
    - borenium compounds Me-TBD-BBN$^+$, ferrocene borenium derivatives responding to the formula

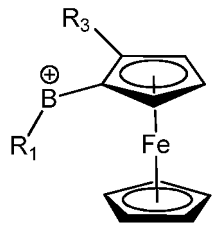

borenium ferrocène

wherein $R^1$ is a phenyl group and $R^3$ is 3,5-dimethylpyridyl;
    - aluminum compounds chosen from among $AlCl_3$, $AlBr_3$, aluminium isopropoxide $Al(O\text{-}i\text{-}Pr)_3$, aluminium ethanoate ($Al(C_2H_3O_2)$), Krossing salt $[Ag(CH_2Cl_2)]\{Al[OC(CF_3)_3]_4\}$, $Li\{Al[OC(CF_3)_3]_4\}$, $Et_2Al^+$;
    - indium compounds chosen from among $InCl_3$, $In(OTf)_3$;
    - iron compounds chosen from $FeCl_3$, $Fe(OTf)_3$;
    - tin compounds chosen from among $SnCl_4$, $Sn(OTf)_2$;
    - phosphorus compounds such as $PCl_3$, $PCl_5$, $POCl_3$;
    - trifluoromethanesulfonate or triflate compounds ($CF_3SO_3^-$) of transition metals and lanthanides chosen from among scandium triflate, ytterbium triflate, yttrium triflate, cerium triflate, samarium triflate, niodinium triflate.

8. Method according to any one of claims 1 to 3 and 7, **characterised in that** the catalyst is chosen from among $BF_3$; $InCl_3$; triphenylcarbenium tetrakis(perfluorophenyl)borate $[(Ph)_3C^+B(C_6F_5)_4^-, B(C_6F_5)_3]$.

9. Method according to any one of claims 1 to 8, **characterised in that** the reaction is carried out under a pressure of one or a mixture of inert gas(es) chosen from among nitrogen and argon, or gases generated by the method, in particular methane and hydrogen, said pressure being comprised between 0.2 and 50 bars, limits included.

10. Method according to any one of claims 1 to 9, **characterised in that** the reaction is achieved in one or a mixture of at least two solvent(s) chosen from among:

    - silyl ethers chosen from among 1,1,1,3,3,3-hexamethyldisiloxane ($(Me_3Si)_2O$), 1,1,1,3,3,3-hexaethyldisiloxane ($(Et_3Si)_2O$);
    - hydrocarbons chosen from among benzene, toluene, pentane and hexane;
    - sulfoxides chosen from among dimethylesulfoxide (DMSO);
    - alkyl halides chosen from among chloroform, methylene chloride, chlorobenzene, dichlorobenzene.

11. Method according to any one of claims 1 to 10, **characterised in that** the mass ratio between the silane compound of formula (I) and lignin is comprised between 0.5 and 6, limits included.

12. Method according to any one of claims 1 to 11, **characterised in that** the quantity of catalyst is 0.001 to 1 equivalent mass, limits included, with respect to the initial mass of the lignin.

13. Use of a method of depolymerising lignin according to any one of claims 1 to 12, in the production of fuels, electronic

components, plastic polymers, rubber, medicines, vitamins, cosmetic products, perfumes, foodstuffs, synthetic threads and fibres, synthetic leathers, glues, pesticides and fertilizers.

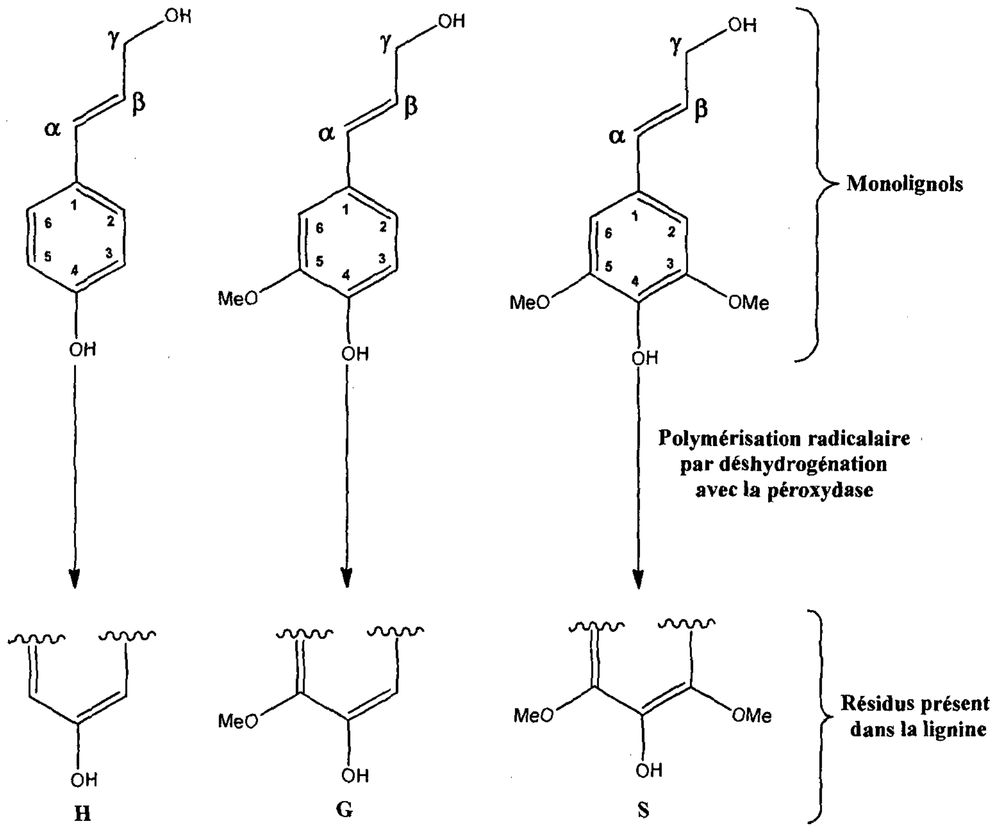
Monolignols
Polymérisation radicalaire
par déshydrogénation
avec la péroxydase
Résidus présent
dans la lignine

H G S

**Figure 1**

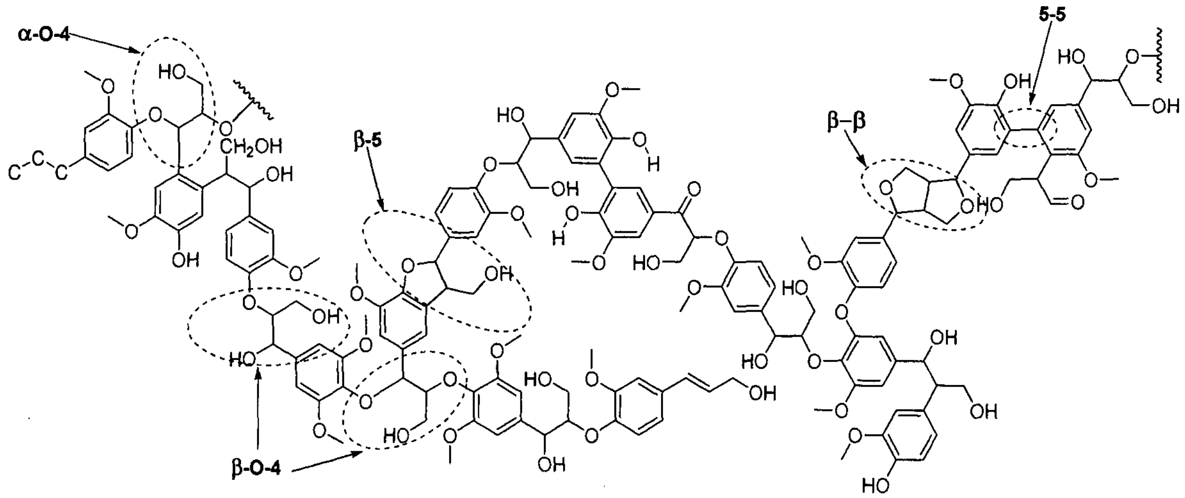

Figure 2

| Liaison | Bois tendre ou Softwood (épicéa) % | Bois dur ou Hardwood (bouleau) % |
|---|---|---|
| **β-O-4** | 46 | 60 |
| **α-O-4** | 6-8 | 6-8 |
| **4-O-5** | 3,5-4 | 6.5 |
| **β-5** | 9-12 | 6 |
| **5-5** | 9,5-11 | 4.5 |
| **β-1** | 7 | 7 |
| **β-β** | 2 | 3 |
| **Autres** | 13 | 5 |

Figure 3

Les types de liaisons les plus abondantes dans la lignine

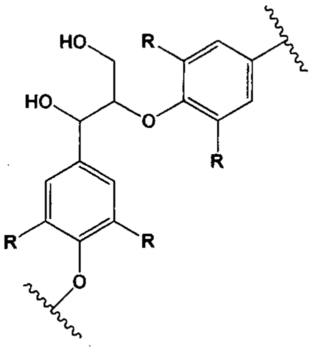

β–O–4

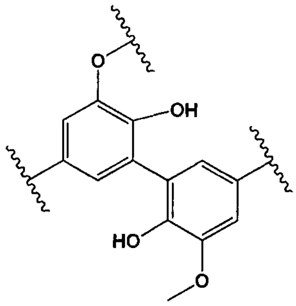

5–5

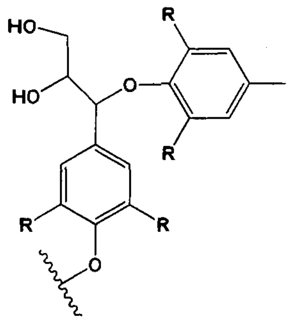

α–O–4

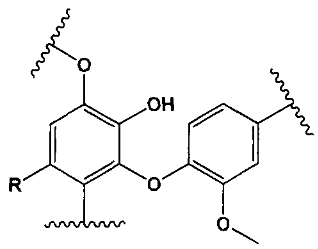

4–O–5

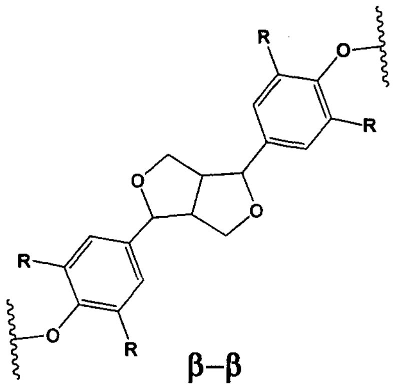

β–β

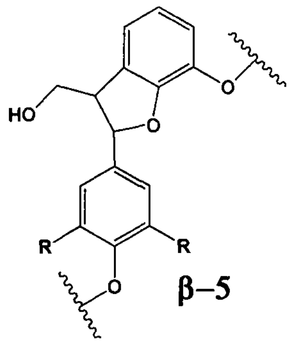

β–5

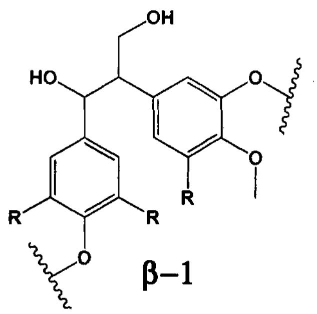

β–1

**Figure 4**

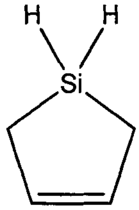

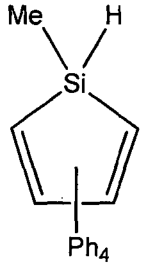

1-silacyclo-3-pentène 1-méthyl-1-hydrido-2,3,4,5-tétraphényl-1-silacyclopentadiène

**Figure 5**

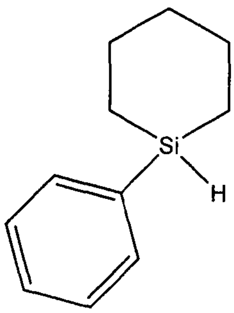

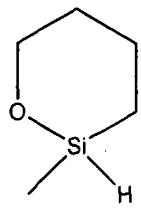

methyl siloxane

1-phenyl-1-silacyclohexane

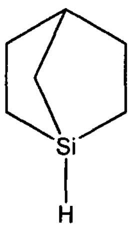

1-sila-bicyclo[2.2.1]heptane

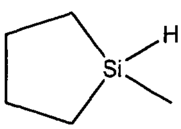

1-methyl-1-silacyclopentane

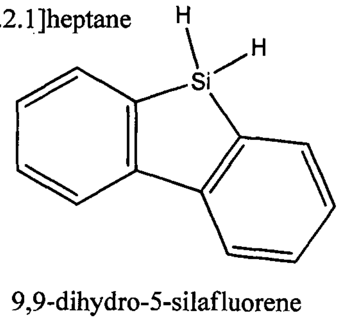

9,9-dihydro-5-silafluorene

**Figure 6**

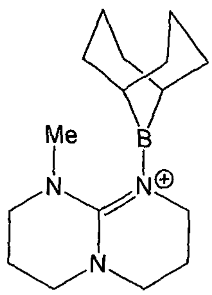
Me-TBD-BBN⁺

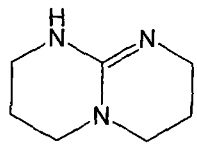
TBD

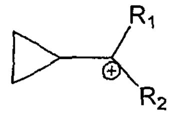
carbocation cyclopropyl

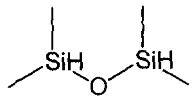
TMDS

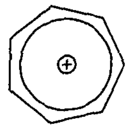
cation tropylium

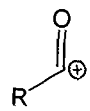
cation acylium

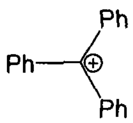
*cation trityl*

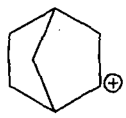
*carbocations norbornyl*

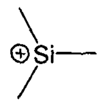
*cation silylium*

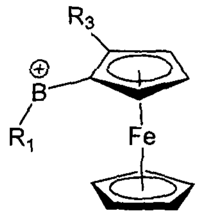
*borenium ferrocène*

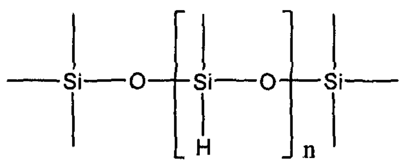
PMHS

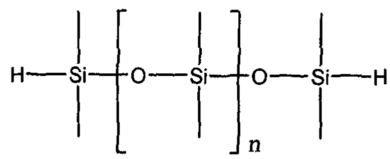
PDMS

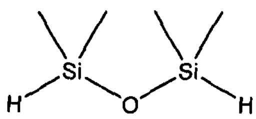
TMDS

**Figure 7**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2011003029 A


### Littérature non-brevet citée dans la description


- **F. G. CALVO-FLORES ; J. A. DOBADO.** *ChemSusChem.,* 2010, vol. 3, 1227-1235
- Methods in Lignin Chemistry. **S. Y. LIN.** Springer Series in Wood Science. Springer, 1992
- **R. VANHOLME ; K. MORREEL ; J. R. W. BOERJAN.** *Curr. Opin. Plant Biol.,* 2008, vol. 11, 278-285
- **F. G. CALVO-FLORES.** *J. A. Dobado, ChemSusChem.,* 2010, vol. 3, 1227-1235
- **E. ADLER.** *Wood Sci. Technol.,* 1977, vol. 11, 169
- **M. P. PANDEY ; C. S. KIM.** *Chem. Eng. Technol.,* 2011, vol. 34, 29
- **J. ZAKZESKI ; P. C. A. BRUIJNINCX ; A. L. JONGERIUS ; B. M. WECKHUYSEN.** *Chem Rev.,* 2010, vol. 110, 3552
- **J. M. NICHOLS ; L. M. BISHOP ; R. G. BERGMAN ; J. A. ELLMAN.** *J. Am. Chem. Soc.,* 2010, vol. 132, 12554-12555
- **A. WU ; B. O. PATRICK ; E. CHUNG ; B. R. JAMES.** *Dalton Trans.,* 2012, vol. 41, 11093
- **T. VOM STEIN ; T. WEIGAND ; C. MERKENS ; JURGEN KLANKERMAYER. ; W. LEITNER.** *ChemCatChem,* 2013, vol. 5, 439-441
- **S. SON ; F. D. TOSTE.** *Angew. Chem, Int, Ed.,* 2010, vol. 49, 3791-3794
- **J. M. W. CHAN ; S. BAUER ; H. SOREK ; S. SREEKUMAR ; K. WANG ; F. D, TOSTE.** *ACS Catal.,* 2013, vol. 3, 1369-1377
- **A. G. SERGEEV ; J. F. HARTWIG.** *Science,* 2011, vol. 332, 439
- **M. NAGY ; K. DAVID ; G. J. P. BRITOVSEK ; A. J. RAGAUSKAS.** *Holzforschung,* 2009, vol. 63, 513
- **L.B. DAVIN, L.B. ; N. G. LEWIS.** *Curr. Opin. Biotechnol.,* 2005, vol. 16, 407-415
- **E. FEGHALI ; T. CANTAT.** *Chem. Commun.,* 2014, vol. 50, 862-865
- **J. ZHENG ; Y. CHEN ; M.A. BROOK.** *Acs Sustainable Chemistry and Engineering,* 03 Juillet 2014, vol. 2, 1983-1991
- **S. SON ; F. D. TOSTE.** *Angew. Chem. Int. Ed.,* 2010, vol. 49, 3791-3794
- **J. J. STEWART ; T. AKIYAMA ; C. CHAPPLE ; J. RALPH ; S. D. MANSFIELD.** *Plant Physiol.,* 2009, vol. 150, 621-635
- **J. H. LORA ; W. G. CLASSER.** *J Polym Environ,* 2002, vol. 10, 39-48
- **BOJAN JANKOVIC.** *Bioresource Technol.,* 2011, vol. 102, 9763-9771
- **J. C. PARAJO ; J. L. ALONSO ; D. VAZQUEZ.** *Bioresource Technology,* 1993, vol. 46, 233-240
- **I. MIRANDA ; H. PEREIRA.** *Holzforschung,* 2002, vol. 56, 85-90
- **A. JOHANSSON ; O. AALTONEN ; P. YLINEN.** *Biomass,* 1987, vol. 13, 45-65
- **X.F. SUN ; R.C. SUN ; P. FOWLER ; M.S, BAIRD.** *Carbohydr. Polym.,* 2004, vol. 55, 379-391
- **P. LIGERO ; A. VEGA ; J.J. VILLAVERDE.** *Bioresource Technol.,* 2010, vol. 101, 3188-3193
- **A. LINDNER ; G. WEGENER.** *J. Wood Chem, Technol.,* 1988, vol. 8, 323-340
- **R. PANISCH ; M. BOITE ; T. MULLER.** *J. Am. Chem. Soc.,* 2006, vol. 128, 9676-9682
- **R. R. NAREDLA ; D. A. KLUMPP.** *Chem. Rev.,* 2013, vol. 113, 6905-6948
- **M. SAUNDERS. ; H. A. JIMENEZ-VAZQUEZ.** *Chem. Rev.,* 1991, vol. 91, 375-397
- **I. GOTTKER-SCHNETMANN ; P. WHITE ; M. BROOKHART.** *J. Am. Chem. Soc.,* 2004, vol. 126, 1804-1811
- **J. YANG ; M. BROOKHART.** *J. Am. Chem. Soc.,* 2007, vol. 129, 12656-12657
- **T. STAHL ; H. F. T. KLARE ; M. OESTREICH.** *J. Am. Chem. Soc.,* 2013, vol. 135, 1248-1251
- **J. CHEN ; R. A. LALANCETTEA ; F. JÄKLE.** *Chem. Commun.,* 2013, vol. 49, 4893-4895
- **I. KROSSING.** *Chem.-Eur. J.,* 2001, vol. 7, 490
- **M. KHANDELWAL ; R. J. WEHMSCHULTE.** *Angew. Chem. Int. Ed.,* 2012, vol. 51, 7323-7326
- **J. H. LORA ; W. G. GLASSER.** *J Polym Environ,* 2002, vol. 10, 39-48
- **X.F. SUN ; R.C. SUN ; P. FOWLER ; M.S. BAIRD.** *Carbohydr. Polym.,* 2004, vol. 55, 379-391

- **A. LINDNER ; G. WEGENER.** *J. Wood Chem. Technol.,* 1988, vol. 8, 323-340
- **H. Q. LAM ; Y. LE BIGOT ; M. DELMAS ; G. AVIGNON.** *Industrial Crops and Products,* 2001, vol. 14, 139-144
- **S. BAUER ; H. SOREK ; V. D. MITCHELL ; A. B. IBÁÑEZ ; D. E. WEMMER.** *J. Agric. Food Chem.,* 2012, vol. 60, 8203-8212
- **K. BARTA ; G. R. WARNER ; E. S. BEACH ; P. T. ANASTAS.** *Green Chem.,* 2014, vol. 16, 191-196
- **S. BAUER ; H. SOREK ; V. D. MITCHELL ; A. B, IBÁÑEZ ; D. E. WEMMER.** *J. Agric. Food Chem.,* 2012, vol. 60, 8203-8212